# NEW EUROPEAN PATENT SPECIFICATION

(11) **EP 2 194 781 B2**
(45) Date of publication and mention of the opposition decision: **24.03.2021**
(45) Mention of the grant of the patent: 21.02.2018
(21) Application number: 08835957.5
(22) Date of filing: 02.10.2008
(51) Int. Cl.: A61K 35/60, A61K 31/198, A61K 31/201, A61K 31/202, A61K 31/22, A61K 31/23, A61K 31/355, A61K 31/375, A61K 33/04, A61K 45/06, A61P 25/28, A23K 20/158, A23K 50/42, A23K 50/48

(54) **COMPOSITIONS AND METHODS FOR ENHANCING COGNITIVE FUNCTION**
ZUSAMMENSETZUNGEN UND VERFAHREN ZUR FÖRDERUNG DER KOGNITIVEN FUNKTION
COMPOSITIONS ET PROCÉDÉS PERMETTANT D'AMÉLIORER LA FONCTION COGNITIVE

(30) Priority: 04.10.2007 US 997665 P
(43) Date of publication of application: 16.06.2010
(73) Proprietor: Société des Produits Nestlé S.A., 1800 Vevey (CH)
(72) Inventor: PAN, Yuanlong, Chesterfield MO 63117 (US); WALDRON, Mark, CH-1073 Savigny (CH); LARSON, Brian, T., Dowling MI 49050 (US)
(74) Representative: Rupp, Christian
(86) International application number: PCT/US2008/011424
(87) International publication number: WO 2009/045481

(56) References cited:
- EP-A1- 1 665 940
- WO-A1-2007/022991
- WO-A1-2009/002165
- WO-A2-99/33355
- WO-A2-03/041701
- WO-A2-2007/041418
- WO-A2-2007/073178
- US-A- 5 780 039
- US-A- 5 977 138
- US-A1- 2002 182 196
- US-A1- 2003 113 701
- US-A1- 2005 002 992
- US-A1- 2007 026 049
- US-A1- 2007 060 651
- US-A1- 2007 179 197
- REGER M A ET AL: "Effect of .beta.-hydroxybutyrate on cognition in memory-impaired adults", NEUROBIOLOGY OF AGING, TARRYTOWN, NY, US, vol. 25, no. 3, 1 March 2004 (2004-03-01), pages 311-314, XP008122713, ISSN: 0197-4580, DOI: DOI:10.1016/S0197-4580(03)00087-3
- MAHER ET AL: "Effects of nutrients on brain function", 1 January 2000 (2000-01-01), THE BIOLOGICAL BASIS FOR MIND BODY INTERACTIONS,, PAGE(S) 187 - 194, XP009150320, * page 192 *
- T Igarashi ET AL: "Nondestructive quantitative determination of docosahexaenoic acid and n-3 fatty acids in fish oils by high-resolution 1H nuclear magnetic resonance spectroscopy.", Journal of the American Oil Chemists' Society, 1 July 2000 (2000-07-01), pages 737-748, XP055271003, DOI: 10.1007/s11746-000-0119-0 Retrieved from the Internet: URL:http://rd.springer.com/content/pdf/10. 1007/s11746-000-0119-0.pdf [retrieved on 2016-05-09]
- Anonymous: "Young At Heart - Dog Longevity & Anti Aging- "Friends for Life Deserve Longer Life"", Internet Archive Wayback Machine, 24 August 2007 (2007-08-24), Retrieved from the Internet: URL:https://web.archive.org/web/2007082407 2240/http://www.k9power.com:80/young_at_he art.php
- Anonymous: "K9 Young at Heart ", Internet Archive Wayback Machine, 24 August 2007 (2007-08-24), Retrieved from the Internet: URL:https://web.archive.org/web/2007082421 4113/http://www.an-nat.com:80/animalhealth _youngheart.html
- Noel et al: "Abstracts: Nonpharmacological and Lifestyle Interventions", , 2005, pages S62, Abs:P-165,
- Paul et al: Indian J Physiol Pharmacol, vol. 49, no. 2, 2005, pages 179-186,
- Taha et al: Neurochem Res, 20 March 2009 (2009-03-20), pages 1-7,
- Pan et al: British J Nutrition, vol. 103, 2010, pages 1746-1754,
- Eid et al: Nutrition & Metabolism, vol. 3, no. 4, 2006, pages 1-10,
- Souci Fachmann Kraut: Food composition and nutrition tables, 2008, pages 980-981,

## Description

### CROSS REFERENCE TO RELATED APPLICATIONS

This application claims priority to U.S. Provisional Application Serial No. 60/997,665 filed October 04, 2007.

### BACKGROUND OF THE INVENTION

### Field of the Invention

The invention relates generally to use of compositions according to the claims for enhancing cognitive function and particularly to use of compositions comprising long chain polyunsaturated fatty acids, nitric oxide releasing compounds, and medium chain triglycerides for enhancing cognitive function in animals.

### Description of Related Art

Aged or aging animals frequently suffer some degree of cognitive impairment. Changes, including decline in cognitive function that progresses with age, and age-related changes in brain morphology and cerebrovascular function are commonly observed, *e.g*., brain aging. Age-related or age-associated cognitive impairment may manifest itself in many ways, *e.g*., short-term memory loss, diminished capacity to learn, diminished rate of learning, diminished attention, diminished motor performance, and/or dementia, among other indicia. In some cases, a specific etiology of such cognitive decline is unknown. In other cases, cognitive impairment results from the onset or progression of recognized diseases, disorders, or syndromes, for example, Alzheimer's Disease (AD). It is known that age-associated cognitive decline is distinct from and can occur independently of AD.

Animal models of cognitive impairment greatly facilitate the study of such conditions including their physiology, neurology, anatomy, and pathology. Dogs are useful model animals that demonstrate age-associated cognitive decline in learning and memory that varies depending on the function of the cognitive task (Adams B *et al.*, 2000a; Chan ADF *et al*., 2002; Su M-Y *et al.*, 1998; and, Tapp PD *et al.,* 2003). While the study of such decline in dogs is useful in its own right because of their role as companion animals, the fact that the observed decline mirrors age-related cognitive declines seen in humans (Adams B *et al.* 2000b) makes the studies even more valuable. Aged dogs develop neuropathology that is related to that seen in both successfully aging humans and patients with AD, such as beta amyloid protein (Cotman CW and Berchtold, 2002; and Cummings BJ *et al*., 1996). However, dogs do not demonstrate every hallmark of AD, in particular, tau-containing neurofibrillar tangles (Dimakopoulos AC *et al.,* 2002) have not been observed. Therefore, the condition in dogs is distinct and referred to as Canine Cognitive Dysfunction Syndrome (CCDS).

Both healthy dogs and unhealthy dogs such as those diagnosed with CCDS may present clinically with progressive cognitive impairment and neuropathological changes (London ED *et al.*, 1983). In addition, aging dogs and those diagnosed with CCDS exhibit various behavioral disorders. For example, they may not respond to their name or familiar commands, may get lost or confused even in familiar surroundings, may no longer greet or respond to their owners or visitors, may exhibit diminished daytime activity, may walk in circles, may shun affection, and may lose bladder or bowel control.

Age-related cognitive decline has been correlated with impaired glucose metabolism. (Finch CE *et al.*, 1997). The primary energy source of the healthy animal or mammalian brain is glucose. Impaired glucose metabolism can diminish the ability of cells to repair and resist oxidative damage. (Munch G *et al.,* 1998). The concomitant neuronal loss and morphological abnormalities appear to contribute to the reduced mental capacity in the aged. Thus, impaired glucose metabolism can produce an energy deficit in the brain, and may result in neuronal loss and morphological changes in the brain. (Hoyer S., 1990).

Alzheimer's patients also exhibit decreased glucose metabolism. Decreased levels of cerebral glucose are evident with positron emission tomography. (Drzezga A *et al*., 2005; and, Small GW *et al.,* 2000). Although the precise mechanisms underlying the decrease in glucose levels and glucose metabolism is not fully understood, neuropathological events such as oxidative stress, neuronal cell death, and decreased levels of acetylcholine, ATP, and cholesterol, have all been correlated with decreased energy and glucose metabolism in the brain. (Swaab *et al*., 1998).

In addition to the effects of changes in glucose metabolism, according to one hypothesis, a reduction in the regional blood flow to the brain contributes to cognitive decline and dementia in humans (Wardlaw JM *et al*., 2003). Regional cerebral blood volume is affected by human age and stage of dementia (Split A *et al.*, 2005; and, Petrella JR *et al.,* 1998). Dogs also experience age-related reduction in regional cerebral metabolic rates for glucose (London ED *et al*., 1983). Dogs exhibit age-dependent changes in regional cerebral blood volume and blood-brain barrier permeability that may be related to changes in brain structure, and neuropathology (Tapp PD *et al*., 2005; and, Su MY, 1998).

Medium chain triglycerides (MCTs) have previously been shown to be useful in the enhancement or preservation of cognitive functions in aging populations. MCTs are composed of fatty acid chains esterified to a glycerol backbone. Ketone bodies produced from MCTs can provide an alternative energy source to supplement the energy deficit in neuronal cells of Alzheimer's patients (Reger MA *et al*., 2004; U.S. Patent Nos. 6,323,237 and 6,316,038). MCTs have also been shown to preserve or improve certain cognitive skills in aging dogs when administered as part of a long-term dietary regimen (WO 2007/070701).

US 2007/0179197 discloses compositions suitable for treatment of individuals with age associated cognitive decline such as Age Associated Memory Impairment or Alzheimer's disease, or Mild Cognitive Impairment. MCTs as used in these compositions are described to preserve learning, attention, motor performance, cerebrovascular function, social behavior, and to increase activity levels, particularly in aging mammals. While, however, disclosing the use of MCTs for the treatment of several disease conditions such as Alzheimer's disease or in dogs, age-associated cognitive decline in learning and memory, US 2007/0179197 clearly fails to mention or suggest the application of LCPUFAs and NORCs in combination with MCTs.

Though advances have been made, there remains a need to develop compositions and methods that improve cognition, particularly in aging humans and other animals. Compositions and methods for the treatment and/or prevention of cognitive impairment are also needed. Such therapies would be useful to improve the overall quality of life for all involved. For companion animals, these therapies would lead to improved owner satisfaction and would improve the owner-companion animal bond.

### SUMMARY OF THE INVENTION

It is, therefore, an object of the present invention to provide compositions useful for enhancing cognitive function in an animal.

It is another object of the present invention to provide uses and compositions therefore for reducing or preventing a decline of social interaction, reducing or preventing age-related behavioral changes, increasing trainability, maintaining optimal brain function, facilitating learning and memory, reducing memory loss, retarding brain aging, preventing or treating strokes, and preventing: or treating dementia in an animal.

It is a further object of the invention to provide articles of manufacture in the form of kits that contain one or more of the compounds useful to produce the composition of the present invention in combination with foods or other ingredients and devices useful for enhancing cognitive function in an animal.

It is another object of the invention to provide a package comprising a composition of the present invention and a label affixed to the package indicating the contents of the package and/or the benefits of administering the composition to an animal.

One or more of these other objects are achieved using novel compositions according to the claims for enhancing cognitive function. Generally, the compositions comprise one or more long chain polyunsaturated fatty acids, nitric oxide releasing compounds, and medium chain triglycerides. The disclosure describes administering the compositions in an amount effective for enhancing cognitive function, particularly to prevent, reduce and/or delay the age-related cognitive decline in an animal.

Other and further objects, features, and advantages of the present invention will be readily apparent to those skilled in the art.

### DETAILED DESCRIPTION OF THE INVENTION

### Definitions

The following abbreviations may be used herein: MCTs, medium chain triglycerides; AA, arachidonic acid; ALA, alpha-linolenic acid; ANOVA, analysis of variance; DHA, docosahexaenoic acid; DPA, docosapentaenoic acid; EPA, eicosapentaenoic acid; LA, linoleic acid; LCPUFA, long chain polyunsaturated fatty acids (as used herein LCPUFA refers to one or more such fatty acids); NO, nitric oxide; NORC, nitric oxide releasing compound or compounds; and L-Arg, L-arginine.

The term "animal" means any animal that could benefit from one or more of the methods of the present invention including enhancing cognitive function; altering cognitive, motor, or behavioral function; reducing or preventing decline of social interaction; reducing or preventing age-related behavioral changes; increasing trainability; maintaining optimal brain function; facilitating learning and memory; and/or reducing memory loss. Generally, the animal is a human, avian, bovine, canine, equine, feline; hicrine, lupine, murine, ovine, and porcine animal. Preferably, the term "animal" means an animal for which an enhancement of cognitive function is desired or would benefit from an improvement in cognitive function. A "companion animal" is any domesticated animal, and includes, without limitation, cats, dogs, rabbits, guinea pigs, ferrets, hamsters, mice, gerbils, horses, cows, goats, sheep, donkeys, pigs, and the like. Preferably, the animal is a human or a companion animal such as a dog or cat.

The term "enhancing cognitive function" means one or more of increasing cognitive, motor, or behavioral function in an animal or preventing, reducing, or delaying a decline in cognitive, motor, or behavioral function in an animal.

The term "medium chain triglycerides" or "MCTs" means any glycerol molecule ester-linked to three fatty acid molecules, each fatty acid molecule having 5-12 carbons. MCTs may be represented by the following general formula (Formula 1): 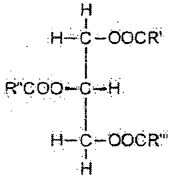 where R', R" and R"' are fatty acids having 5-12 carbons in the carbon backbone esterified to a glycerol backbone. The lipids of the invention may be prepared by any process known in the art, such as direct esterification, rearrangement, fractionation, transesterification, or the like. For example, the lipids may be prepared by the rearrangement of a vegetable oil such as coconut oil. The length and distribution of the chain length may vary depending on the source oil. For example, MCTs containing 1-10% C6, 30-60% C8, 30-60% C10, 1-10% C10 are commonly derived from palm and coconut oils. MCTs containing greater than about 95% C8 at R', R" and R"' can be made by semi-synthetic esterification of octanoic acid to glycerin. Also useful herein are mixtures comprising MCTs with about 50% total C8 and/or about 50% total C10. Commercial sources for the foregoing MCT compositions are available and known to the skilled artisan. Such MCTs behave similarly and are encompassed within the term MCTs as used herein.

The term "long chain polyunsaturated fatty acids" or "LCPUFA" means any one or more monocarboxylic acids having at least 20 carbon atoms and at least two double bonds. Examples of LCPUFA include (ri-6) fatty acids, such as arachidonic acid (AA), and (n-3) fatty acids, such as eicosapentaenoic acid (EPA), docosapentaenoic acid (DPA) and docosahexaenoic acid (DHA).

The term "fish oil" means a fatty or oily extract, relatively rich in LCPUFA, whether crude or purified, obtained from a sea animal, preferably a cold-water fish such as, but not limited to, salmon, tuna, mackerel, herring, sea bass, striped bass, halibut, catfish, and sardines, as well as shark, shrimp, and clams, or any combination thereof. Fish oil is generally a term of art used by ingredient suppliers and encompasses a range or products of varying PUFA content and purity.

The term "nitric oxide releasing compounds" or "NORC" means any compound or compounds that cause or can result in the release of nitric oxide in an animal. Examples of such compounds include L-arginine, L-arginine-containing peptides and proteins, and analogs or derivatives thereof that are known or determined to release nitric oxide, such as arginine alpha-ketoglutarate, GEA 3175, sodium nitroprusside, glyceryl trinitrate, *S-*nitroso-*N*-acetyl-penicillamine, nitroglycerin, *S*-NO-glutathione, NO-conjugated non-steroidal anti-inflammatory drugs (e.g., NO-naproxen, NO-aspirin, NO-ibuprofen, NO-Diclofenac, NO-Flurbiprofen, and NO-Ketoprofen), NO-releasing compound-7, NO-releasing compound-5, NO-releasing compound-12, WO-releasing compound-18, diazenium diolates and derivatives thereof, diethylamine NONOate, and any organic or inorganic compound, biomolecule, or analog, homolog, conjugate, or derivative thereof that causes the release of nitric oxide, particularly "free" NO, in an animal

The term "effective amount" means an amount of a compound, material, composition, medicament, or other material that is effective to achieve a particular biological result. Such results include, but are not limited to, one or more of the following: enhancing cognitive function, increasing daytime activity, improving learning (either the rate or ease of learning), improving attention, improving social behavior, improving motor performance, and/or improving cerebrovascular function, particularly in aging animals. In various embodiments, "effective amount" refers to an amount suitable to prevent a decline in any one or more of the above qualities, or, in certain embodiments, to improve any one or more of the above qualities, for example, cognitive function or performance, learning rate or ability, problem solving ability, attention span and ability to focus on a task or problem, motor function or performance, social behavior, and the like. In other embodiments, an effective amount is suitable to reduce either the extent or rate of decline in an animal's cognitive skills or functioning, and/or the effective amount is suitable to delay the onset of such decline. Such effectiveness may be achieved, for example, by administering the compositions of the present invention to an animal or to a population of animals. Preferably the prevention, reduction, or delay of such a decline, or the improvement in an individual or population is relative to a cohort, *e.g.*, a control animal or a cohort population that has not received the treatment, or been administered the composition or medicament.

The term "cognitive function" refers to the special, normal, or proper physiologic activity of the brain, including one or more of the following: mental stability, memory/recall abilities, problem solving abilities, reasoning abilities, thinking abilities, judging abilities, ability to discriminate or make choices, capacity for learning, ease of learning, perception, intuition, attention, and awareness. "Enhanced cognitive function" or "improved cognitive function" refers to any improvement in the special, normal, or proper physiologic activity of the brain, including one or more of the following: mental stability, memory/recall abilities, problem solving abilities, reasoning abilities, thinking abilities, judging abilities, ability to discriminate or make choices, capacity for learning, ease of learning, perception, intuition, attention, and awareness, as measured by any means suitable in the art.

The term "behavior" means anything that an animal does in response or reaction to a given stimulation or set of conditions. "Enhanced behavior" or "improved behavior" means any improvement in anything that an animal does in response or reaction to a given stimulation or set of conditions. "Behavior" is used synonymously with "behavioral function" herein.

The term "motor function" or "motor performance" means the biological activity of the tissues that affect or produce movement in an animal. Such tissue include, without limitation, muscles and motor neurons. "Enhanced motor performance (or function)" or "improved motor performance (or function)" refers to any improvement in the biological activity of the tissues that affect or produce movement in an animal.

The term "decline" of any of the foregoing categories or specific types of qualities or functions in an individual (phenotypes) is generally the opposite of an improvement or enhancement in the quality or function. An "effective amount" of a composition may be an amount required to prevent decline altogether or to substantially prevent decline ("prevent" decline), to reduce the extent or rate of decline ("reduce" decline) over any time course or at any time point, or delay the onset, extent, or progression of a decline ("delay" a decline). Prevention, reduction, or delay of "decline" is frequently a more useful comparative basis when working with non-diseased aging animals (*e.g*., "healthy aging animals"). Prevention, reduction, and delay can be considered relative to a control or cohort which does not receive the treatment, for example, the diet or supplement of interest. Prevention, reduction, or delay of either the onset of a detrimental quality or condition, or of the rate of decline in a particular function can be measured and considered on an individual basis, or in some embodiments on a population basis. The net effect of preventing, reducing, or delaying decline is to have less decrease in cognitive, motor, or behavioral functioning per unit time, or at a given end point. In other words, ideally, for an individual or in a population, cognitive, motor, and behavioral functioning is maintained at the highest possible level for the longest possible time. Thus, it is not required that there be a net increase in cognitive, motor, or behavioral function for any embodiment. For purposes herein, an individual can be compared to a control individual, group, or population. A population can likewise be compared to an actual individual, to normalized measurements for an individual, or to a group or population, as is useful.

The term "aging" means being of advanced age such that the animal has exceeded 50% of the average lifespan for its particular species and/or breed within a species. For example, if the average lifespan for a given breed of dog is 10 years, then a dog within that breed greater than 5 years old would be considered "aging" for purposes herein. "Healthy aging animals" are those with no known diseases, particularly diseases relating to loss of cognitive impairment such as might confound the results. In studies using healthy aging animals, cohort animals are preferably also healthy aging animals, although other healthy animals with suitable cognitive, motor, or behavioral functioning may be suitable for use as comparative specimens. If animals with specific disease diagnoses, or cognitive, motor, or behavioral limitations are used, then the cohort animals should include animals that are similarly diagnosed, or which present with similar indicia of the disease or cognitive, motor, or behavioral limitation.

The term "food" or "food product" or "food composition" means a product or composition that is intended for ingestion by an animal, including a human, and provides nutrition to the animal.

As used herein, a "food product formulated for human consumption" is any composition specifically intended for ingestion by a human being. The term "pet food" or "pet food composition" means a composition intended for consumption by animals, preferably by companion animals. A "complete and nutritionally balanced pet food" is one that contains all known required nutrients for the intended recipient or consumer of the food, in appropriate amounts and proportions, based for example on recommendations of recognized authorities in the field of companion animal nutrition. Such foods are therefore capable of serving as a sole source of dietary intake to maintain life or promote production, without the addition of supplemental nutritional sources. Nutritionally balanced pet food compositions are widely known and used in the art. The term includes any food, feed, snack, food supplement, treat, meal substitute, or meal replacement, whether intended for a human or another animal. Animal food includes food or feed intended for any domesticated or wild species. In preferred embodiments, a food for an animal represents a nutritionally complete food composition, *e.g*., a pelleted, extruded, or dry food. Examples of such animal foods include extruded pet foods, such as foods for dogs and cats.

The term "dietary supplement" means a product that is intended to be ingested in addition to the normal animal diet. Dietary supplements may be in any form, *e.g*., solid, liquid, gel, tablets, capsules, powder, and the like. Preferably they are provided in convenient dosage forms. In some embodiments they are provided in bulk consumer packages such as bulk powders, liquids, gels, or oils. In other embodiments, supplements are provided in bulk quantities to be included in other food items such as snacks, treats, supplement bars, beverages and the like.

The term "long-term administration" means periods of repeated administration or consumption in excess of one month. Periods of longer than two, three, or four months are preferred for certain embodiments. Also preferred are more extended periods that include longer than 5, 6, 7, 8, 9, or 10 months. Periods in excess of 11 months or 1 year are also preferred. Longer term use extending over 1, 2, 3, or more years are included in the invention. For certain aging animals, the animal will continue consuming on a regular basis for the remainder of its life. Sometimes this is referred to as consumption for "extended" periods.

The term "regular basis" means at least monthly dosing with the compositions or consumption of the compositions, more preferably weekly dosing. More frequent dosing or consumption, such as twice or three times weekly, is preferred in certain embodiments. Still more preferred are regimens that comprise at least once daily consumption. The skilled artisan will appreciate that the blood level of a compound or certain metabolites of that compound or which result after the consumption of that compound, may be a useful tool for assessing or determining dosing frequency. For example, for determining dosage or dosage frequency for compositions comprising MCTs, the blood concentration of ketone bodies, or a specific ketone body may provide useful information. A frequency, regardless of whether expressly exemplified herein, that allows maintenance of a desired blood level of the measured compound within acceptable ranges is useful herein. The skilled artisan will appreciate that dosing frequency will be a function of the composition that is being consumed or administered, and some compositions may require more or less frequent administration to maintain a desired blood level of the measured compound (*e.g.*, a ketone body).

The term "oral administration" or "orally administering" means that the animal ingests, or a human is directed to feed, or does feed, the animal one or more of the compositions described herein. Wherein a human is directed to feed the composition, such direction may be that which instructs and/or informs the human that use of the composition may and/or will provide the referenced benefit, for example, enhancing cognitive function, improving liver function, increasing daytime activity, improving learning, improving attention, improving social behavior, improving motor performance, and/or improving cerebrovascular function, or preventing, reducing, or delaying a decline in such foregoing functions or qualities. Such direction may be oral direction (*e.g.*, through oral instruction from, for example, a physician, veterinarian, or other health professional, or radio or television media (*i.e.*, advertisement), or written direction (*e.g.,* through written direction from, for example, a physician, veterinarian, or other health professional (*e.g.,* prescriptions), sales professional or organization (*e.g.*, through, for example, marketing brochures, pamphlets, or other instructive paraphernalia), written media (*e.g.*, internet, electronic mail, website, or other computer-retated media), and/or packaging associated with the composition (*e.g.*, a label present on a container holding the composition), or a combination thereof (*e.g.*, label or package insert with directions to access a website for more information).

The term "cognitive drugs" means any compound, composition, or drug useful for affecting cognitive function, *e.g.*, monoamine oxidase B inhibitors such as selegiline; vasodilators such as nicerogoline and vinpocetine; phosphatidylserine; propentofyline; anticholinesterases (cholinesterase inhibitors) such as tacrine, galantamine, rivastigmine, vinpocetine, donepezil (ARICEPT® (donepezil hydrochloride)), metrifonate, and physostigmine; lecithin; choline cholinomimetics such as milameline and xanomeline; ionotropic N-methyl-D-aspartate (NMDA) receptor antagonists such as memantine; anti-inflammatory drugs such as prednisolone, diclofenac, indomethacin, propentofyline, naproxen, rofecoxin, ibruprofen and suldinac; metal chelating agents such as cliquinol; *Ginkgo biloba*; bisphosophonates; selective oestrogen receptor modulators such as raloxifene and estrogen; beta and gamma secretase inhibitors; cholesterol-lowering drugs such as statins; calcitonin; risedronate; alendronate; and combinations thereof.

The term "in conjunction" means that a composition comprising LCPUFA, NORC, and MCTs, a food composition, cognitive drug, or other compound or composition of the present invention are administered to an animal (1) together in a food composition or (2) separately at the same or different frequency using the same or different administration routes at about the same time or periodically. "Periodically" means that the agent is administered on a dosage schedule acceptable for a specific agent and that the food is fed to an animal routinely as appropriate for the particular animal. "About the same time" generally means that the food and agent are administered at the same time or within about 72 hours of each other. "In conjunction" specifically includes administration schemes wherein a cognitive drug is administered for a prescribed period and the compositions comprising LCPUFA, NORC, and MCTs are administered indefinitely.

The term "individual" when referring to an animal means an individual animal of any species or kind

The term "microorganism" encompasses at least bacteria, molds and other fungi, and yeasts. Probiotics are beneficial microorganisms that can survive or even multiply and thrive in the gastrointestinal tract of an animal. Probiotics can contribute to the overall health of an animal generally and particularly to the gastrointestinal health of the animal.

The term "single package" means that the components of a kit are physically associated, in or with one or more containers, and considered a unit for manufacture, distribution, sale, or use. Containers include, but are not limited to, bags, boxes or cartons, bottles, packages of any type or design or material, over-wrap, shrink-wrap, affixed components (*e.g*., stapled, adhered, or the like), or combinations of any of the foregoing. For example, a single package kit may provide containers of individual compositions and/or food compositions physically associated such that they are considered a unit for manufacture, distribution, sale, or use.

The term "virtual package" means that the components of a kit are associated by directions on one or more physical or virtual kit components instructing the user how to obtain the other components, *e.g.,* in a bag or other container containing one component and directions instructing the user to go to a website, contact a recorded message or a fax-back service, view a visual message, or contact a caregiver or instructor to obtain, for example, instructions on how to use the kit, or safety or technical information about one or more components of a kit. Examples of information that can be provided as part of a virtual kit include instructions for use; safety information such as material safety data sheets; poison control information; information on potential adverse reactions; clinical study results; dietary information such as food composition or caloric composition; general information on cognitive, behavioral, or motor function; diseases that effect cognitive, behavioral, or motor function; treating cognitive, behavioral, or motor function; or general information on treatment or preservation of cognitive, behavioral, or motor function; self-help relating to cognitive, behavioral, or motor function; caregiver information for those caring for animals with cognitive, behavioral, or motor function challenges; and use, benefits, and potential side-effects or counter-indications for cognitive drugs.

All percentages expressed herein are by weight of the composition on a dry matter basis unless specifically stated otherwise. The skilled artisan: will appreciate that the term "dry matter basis" means that an ingredient's concentration or percentage in a composition is measured or determined after any free moisture in the composition has been removed.

As used throughout, ranges are used herein in shorthand, so as to avoid having to set out at length and describe each and every value within the range. Any appropriate value within the range can be selected, where appropriate, as the upper value, lower value, or the terminus of the range.

As used herein and in the appended claims, the singular form of a word includes the plural, and vice versa, unless the context clearly dictates otherwise. Thus, the references "a", "an", and "the" are generally inclusive of the plurals of the respective terms. For example, reference to "a puppy", "a method", or "a food" includes a plurality of such "puppies", "methods", or "foods". Reference herein, for example to "an antioxidant" includes a plurality of such antioxidants, whereas reference to "pieces" includes a single piece. Similarly, the words "comprise", "comprises", and "comprising" are to be interpreted inclusively rather than exclusively. Likewise the terms "include", "including" and "or" should all be construed to be inclusive, unless such a construction is clearly prohibited from the context. Where used herein the term "examples," particularly when followed by a listing of terms is merely exemplary and illustrative, and should not be deemed to be exclusive or comprehensive.

The methods and compositions and other advances disclosed here are not limited to particular methodology, protocols, and reagents described herein because, as the skilled artisan will appreciate, they may vary. Further, the terminology used herein is for the purpose of describing particular embodiments only, and is not intended to, and does hot, limit the scope of that which is disclosed or claimed.

Unless defined otherwise, all technical and scientific terms, terms of art, and acronyms used herein have the meanings commonly understood by one of ordinary skill in the art in the field(s) of the invention, or in the field(s) where the term is used. Although any compositions, methods, articles of manufacture, or other means or materials similar or equivalent to those described herein can be used in the practice of the present invention, the preferred compositions, methods, articles of manufacture, or other means or materials are described herein.

The discussion of references is referred to herein intended merely to summarize the assertions made therein. No admission is made that any such patents, patent applications, publications or references, or any portion thereof, are relevant, material, or prior art. The right to challenge the accuracy and pertinence of any assertion of such patents, patent applications, publications, and other references as relevant, material, or prior art is specifically reserved. Full citations for publications not cited fully within the specification are set forth at the end of the specification.

In one aspect, the invention as claimed provides compositions and uses suitable for enhancing cognitive function in an animal. The compositions comprise one or more long chain polyunsaturated fatty acids (LCPUFA), one or more medium chain triglycerides (MCTs), and one or more nitric oxide releasing compounds (NORC) in an amount effective for enhancing cognitive function in an animal. The compositions further comprise one or more B vitamins, one or more antioxidants, or combinations thereof. The invention is based upon the discovery that compositions comprising LCPUFA, NORC, and MCTs, with B vitamins and antioxidants, are effective for enhancing cognitive function in animals. The compositions are useful for affecting one or more cognitive, motor, or behavioral functions in animals regardless of health or age, *e.g.,* juvenile, adult, or senior animals. The compositions increase one or more cognitive, motor, or behavioral functions in animals, including healthy animals of all ages or animals that are susceptible to or suffering from a decline in cognitive function brought about by the aging process or by disease. Similarly, the compositions prevent, reduce, or delay a decline in cognitive function in animals, particularly aging animals susceptible to or suffering from a decline in cognitive function brought about by the aging process or by disease. The compositions are particularly effective for reducing or delaying the effects of age-related and disease-related cognitive decline in humans and companion animals, particularly dogs and cats. The compositions are also useful for enhancing cognitive function when cognitive decline is caused by changes in brain function, particularly brain aging, or damage from disease, particularly strokes.

The LCPUFA can be any LCPUFA suitable for administration to an animal. LCPUFAs can be obtained from any suitable source, synthetic or natural. Preferred sources of LCPUFA are natural sources of such fatty acids and include, without limitation, primrose; dark green vegetables such as spinach; algae and blue-green algae such as spirulina; plant seeds and oils from plants such as flax, canola, soybean, walnut, pumpkin, safflower, sesame, wheat germ, sunflower, com, and hemp; and fish such as salmon, tuna, mackerel, herring, sea bass, striped bass, halibut, catfish, sardines, shark, shrimp, and clams; and the extracted oils of any one or more of the foregoing. The LCPUFA may also be synthetic, and as such may be produced according to any means suitable in the art, from any suitable starting material. It is to be understood the LCPUFA may comprise a blend of any one or more LCPUFA from any one or more sources, such as those exemplified above, whether natural or synthetic.

The MCTs can be any MCT suitable for administration to an animal. MCTs can be obtained from any suitable source, synthetic or natural. Examples of natural sources of MCT include plant sources such as coconuts and coconut oil, palm kernels and palm kernel oils, and animal sources such as milk from any of a variety of species. In certain embodiments, the LCPUFA comprise one or more (n-3) fatty acids such as ALA, EPA, DPA and DHA. In certain embodiments, the NORC comprise one or more of L-Arg and nitric oxide-releasing derivatives thereof. In preferred embodiments, the MCT comprise a compound having the structure shown in Formula 1 wherein the R', R", and R"' esterified to the glycerol backbone are each independent fatty acids having 5-12 carbons. In certain embodiments, greater than about 95% of the R', R", and R"' are 8 carbons in length. The remaining R', R", and R"' are 6-carbon or 10-carbon fatty acids in some embodiments.. In other embodiments, greater than at least or about 30, 40, or 50% of R', R", and R'" are C8, and/or greater than at least or about 30, 40, or 50% of R', R", and R"' are C10. In one embodiment, about 50% of the R', R", and R'" are C8 and about 50% of R', R", and R"' are C10.

The NORCs can be any NORC suitable for administration to an animal. NORCs can be obtained from any suitable source, synthetic or natural. In various embodiments, the NORC comprises arginine. Presently preferred sources of arginine include, without limitation, animal and plant proteins. Examples of plants considered rich in arginine content and suitable for use herein include, but are not limited to, legumes such as soy, lupins, and carob; grains such as wheat and rice; and fruits such as grapes. Seeds and nuts of plants such as cacao and peanut are also considered rich in arginine content and are therefore useful herein. Some examples of suitable animal proteins considered rich in arginine content are poultry and fish products. The NORC can also be synthetically produced, according to any suitable means in the art. As with LCPUFA, the NORC content of any composition disclosed herein can include a blend of any natural or synthetic NORC. Both LCPUFA and NORC, whether natural or synthetic, can be obtained directly or provided by a commercial source.

In one aspect, the compositions further comprise one or more B vitamins, one or more antioxidants, or combinations thereof. The B vitamins can be any B vitamin suitable for administration to an animal. B vitamins include vitamins B1 (thiamine), B2 (riboflavin), B3 (aka P or PP) (niacin, including nicotinic acid and/or nicotinamide), B5 (pantothenic acid), B6 (pyridoxine), B7 (aka H) (biotin), B8 (myo-inositol), B9 (aka M or B-c) (folic acid), B12 (cobalamin), or salts, conjugates, or derivatives thereof recognized of found to have B vitamin activity. Combinations of any of the foregoing are also useful herein and are sometimes referred to herein as "mixtures" of B vitamins. Since the vitamin requirements vary for different species, not all of the listed compounds are deemed vitamins for all species. For example, since it is known that myo-inositol can be synthesized by humans, it is no longer deemed a vitamin, as it is not required for adequate human nutrition. The antioxidants can be any antioxidant suitable for administration to an animal. Antioxidants arc well known in the art, particularly the art of food technology and food formulation. Natural antioxidant compounds include vitamins (such as A, C and E, and derivative, conjugates, or analogs thereof), as well as plant extracts, including extracts from fruit, vegetables, herbs, seeds, and other types and/or parts of plants. Compounds such as α-lipoic acid, chlorophyll and derivatives thereof, glutathione, ubiquinols (*e.g.,* coenzyme Q10), carotenoids (*e.g.,* lycopene), flavonoids, phenolic acids and polyphenols, and pycnogenol are known to be excellent antioxidants. Some examples of plant sources of antioxidants include those from fruits such as berries (cherry, blackberry, strawberry, raspberry, crowberry, blueberry, bilberry/wild blueberry, black currant), pomegranate, grape, orange, plum, pineapple, kiwi fruit, and grapefruit; those from vegetables including kale, chili pepper, red cabbage, peppers, parsley, artichoke, Brussels sprouts, spinach, lemon, ginger, garlic, and red beets; those from dry fruits like apricots, prunes, and dates; from legumes including broad beans, pinto beans, and soybeans. Also nuts and seeds such as pecans, walnuts, hazelnuts, ground nut, and sunflower seeds; cereals such as barley, millet, oats, and corn. Many natural antioxidants are also available from a wide variety of spices including cloves, cinnamon, rosemary, and oregano. Less widely known sources of antioxidants include *Ginkgo biloba,* and tropical plants such as uyaku, and carica papaya. Antioxidant properties of various teas and green tea, as well as fermented products such as red wine, have become of great interest in recent years and such would be suitable for use herein. Selenium is an excellent oxygen scavenger and works well, especially with vitamin or related tocopherol compounds. Synthetic dietary antioxidants include butylated hydroxyanisole (BHA) and butylated hydroxytoluene (BHT) which are commonly used in food products. Any of the foregoing, alone or in combination, are suited for use herein, as are combinations of natural and synthetic antioxidants.

The compositions comprise LCPUFA, NORC, and MCTs, and B vitamins and antioxidants in the composition, in an amount effective for enhancing cognitive function. Generally, the compositions comprise from about 0.1% to about 30% LCPUFA, preferably from about 1 to about 15% LCPUFA; from about 1 to about 10% NORC; and from about 1% to about 20% MCTs, preferably from about 1 to about 12% MCTs, most preferably from about 1 to about 8% MCTs. In the composition, the B vitamins comprise from about 0.1 to 40 times, preferably from about 1 to 20 times the RDA, preferably from about 1 to 10 times the RDA, and antioxidants comprise from about 0.1 to 25 times, preferably from about 0.01 to 15 times the RDA, most preferably from about 0.01 to 5 times the RDA, most preferably from about 0.01 to 2 times the RDA. In one embodiment, the compositions comprise LCPUFA, NORC, and MCTs, and a mixture of one or more B vitamins and one or more antioxidants in such amounts. In one embodiment, the composition comprises from about 10g to about 30g of MCT, from about 0.5g to about 10g LCPUFA, and from about 0.5g to about 10g NORC, with the RDA for B vitamins and antioxidants.

The compositions may further comprise substances such as minerals, other vitamins, salts, functional additives including, for example, palatants, colorants, emulsifiers, antimicrobial or other preservatives. Minerals that may be useful in such compositions include, for example, calcium, phosphorous, potassium, sodium, iron, chloride, boron, copper, zinc, magnesium, manganese, iodine, selenium and the like. Examples of additional vitamins useful herein include such fat soluble vitamins as A, D, E, and K. Inulin, amino acids, enzymes, coenzymes, and the like may be useful to include in various embodiments.

In one embodiment, the compositions are food compositions, including human and pet food compositions. Such compositions include foods intended to supply the necessary dietary requirements for an animal, animal treats (*e.g.,* biscuits), or dietary supplements. The compositions may be a dry composition (*e.g.,* kibble), semi-moist composition, wet composition, or any mixture thereof. In another embodiment, the composition is a dietary supplement such as a gravy, drinking water, beverage, yogurt, powder, granule, paste, suspension, chew, morsel, treat, snack, pellet, pill, capsule, tablet, or any other suitable delivery form. The dietary supplement can comprise a high concentration of the LCPUFA, NORC, and MCTs, and optional B vitamins and antioxidants. This permits the supplement to be administered to the animal in small amounts, or in the alternative, can be diluted before administration to an animal. The dietary supplement may require admixing, or preferably be admixed with water or other diluent prior to administration to the animal.

In one embodiment, the compositions are refrigerated or frozen compositions. In another embodiment, the LCPUFA, NORC, and MCTs are pre-blended with the other components to provide the beneficial amounts needed. In yet other embodiments, the LCPUFA, NORC, and MCTs are used to coat a food, snack, pet food composition, or pet treat. In one embodiment, the LCPUFA, NORC, and MCTs are added to the composition just prior to offering it to the animal, *e.g.,* using a sprinkled powder or a mix. Such compositions can further comprise B vitamins and/or antioxidants,

The compositions can optionally comprise one or more supplementary substances that promote or sustain general health. Preferred substances may be associated with improved mental health or enhanced cognitive function or may be substances that inhibit, delay, or decrease loss of cognitive function, *e.g.,* herbs or plants that enhance cognitive function.

In various embodiments, pet food or pet treat compositions comprise from about 15% to about 50% crude protein. The crude protein material may comprise vegetable proteins such as soybean meal, soy protein concentrate, corn gluten meal, wheat gluten, cottonseed, and peanut meal, or animal proteins such as casein, albumin, and meat protein. Examples of meat protein useful herein include pork, lamb, equine, poultry, fish, and mixtures thereof.

The compositions may further comprise from about 5% to about 40% fat. The compositions may further comprise a source of carbohydrate. The compositions may comprise from about 15% to about 60% carbohydrate. Examples of such carbohydrates include grains or cereals such as rice, corn, milo, sorghum, alfalfa, barley, soybeans, canola, oats, wheat, and mixtures thereof. The compositions may also optionally comprise other materials such as dried whey and other dairy by-products.

In some embodiments, the ash content of the composition ranges from less than 1% to about 15%, preferably from about 5% to about 10%.

The moisture content can vary depending on the nature of the composition. In a preferred embodiment, the composition is a complete and nutritionally balanced pet food. In this embodiment, the pet food may be a "wet food", "dry food", or food of intermediate moisture content. "Wet food" describes pet food that is typically sold in cans or foil bags, and has a moisture content typically in the range of about 70% to about 90%. "Dry food" describes pet food which is of a similar composition to wet food, but contains a limited moisture content, typically in the range of about 5% to about 15% or 20%, and therefore is presented, for example, as small biscuit-tike kibbles. In one presently preferred embodiment, the compositions have moisture content from about 5% to about 20%. Dry food products include a variety of foods of various moisture contents, such that they are relatively shelf-stable and resistant to microbial or fungal deterioration or contamination. Also preferred are dry food compositions which are extruded food products, such as pet foods, or snack foods for either humans or companion animals.

The compositions may also comprise one or more fiber sources. The term "fiber" includes all sources of "bulk" in the food whether digestible or indigestible, soluble or insoluble, fermentable or nonfermentable. Preferred fibers are from plant sources such as marine plants but microbial sources of fiber may also be used. A variety of soluble or insoluble fibers may be utilized, as will be known to those of ordinary skill in the art. The fiber source can be beet pulp (from sugar beet), gum arabic, gum talha, psyllium, rice bran, carob bean gum, citrus pulp, pectin, fructooligosaccharide, short chain oligofructose, mannanoligofructose, soy fiber, arabinogalactan, galactooligosaccharide, arabinoxylan, or mixtures thereof.

Alternatively, the fiber source can be a fermentable fiber. Fermentable fiber has previously been described to provide a benefit to the immune system of a companion animal. Fermentable fiber or other compositions known to skilled artisans that provide a prebiotic to enhance the growth of probiotics within the intestine may also be incorporated into the composition to aid in the enhancement of the benefit provided by the present invention to the immune system of an animal.

In other embodiments, the compositions further comprise prebiotics or probiotics. Probiotics are live microorganisms that have a beneficial effect in the prevention and treatment of specific medical conditions when ingested. Probiotics are believed to exert biological effects through a phenomenon known as colonization resistance. The probiotics facilitate a process whereby the indigenous anaerobic flora limits the concentration of potentially harmful (mostly aerobic) bacteria in the digestive tract. Other modes of action, such as supplying enzymes or influencing enzyme activity in the gastrointestinal tract, may also account for some of the other functions that have been attributed to probiotics. Prebiotics are nondigestible food ingredients that beneficially affect host health by selectively stimulating the growth and/or activity of bacteria in the colon. Prebiotics include fructooligosaccharides (FOS), xylooligosaccharides (XOS), galactooligosaccharides (GOS), and mannooligosaccharides (typically for non-human foods such as petfoods). The prebiotic, fructooligosaccharide (FOS) is found naturally in many foods such as wheat, onions, bananas, honey, garlic, and leeks. FOS can also be isolated from chicory root or synthesized enzymatically from sucrose. FOS fermentation in the colon results in a large number of physiologic effects including increasing the numbers of bifidobacteria in the colon, increasing calcium absorption, increasing fecal weight, shortening of gastrointestinal transit time, and possibly lowering blood lipid levels. The increase in bifidobacteria has been assumed to benefit human health by producing compounds to inhibit potential pathogens, by reducing blood ammonia levels, and by producing vitamins and digestive enzymes. Probiotic bacteria such as Lactobacilli or Bifidobacteria are believed to positively affect the immune response by improving the intestinal microbial balance leading to enhanced antibody production and phagocytic (devouring or killing) activity of white blood cells. Bifidobacterium lactis could be an effective probiotic dietary supplement for enhancing some aspects of cellular immunity in the elderly. Probiotics enhance systemic cellular immune responses and may be useful as a dietary supplement to boost natural immunity in otherwise healthy adults. Probiotics include many types of bacteria but generally are selected from four genera of bacteria: *Lactobacillus acidophillus. Bifidobacteria, Lactococcus,* and *Pediococcus*. Beneficial species include *Enterococcus* and *Saccharomyces* species. The amount of probiotics and prebiotics to be administered to the animal is determined by the skilled artisan based upon the type and nature of the prebiotic and probiotic and the type and nature of the animal, *e.g.,* the age, weight, general health, sex, extent of microbial depletion, presence of harmful bacteria, and diet of the animal. Generally, probiotics are administered to the animal in amounts of from about one to about twenty billion colony forming units (CFUs) per day for the healthy maintenance of intestinal microflora, preferably from about 5 billion to about 10 billion live bacteria per day. Generally, prebiotics are administered in amounts sufficient to positively stimulate the healthy microflora in the gut and cause these "good" bacteria to reproduce. Typical amounts are from about one to about 10 grams per serving or from about 5% to about 40% of the recommended daily dietary fiber for an animal. The probiotics and prebiotics can be made part of the composition by any suitable means. Generally, the agents are mixed with the composition or applied to the surface of the composition, *e.g*., by sprinkling or spraying. When the agents are part of a kit, the agents can be admixed with other materials or in their own package.

The compositions and dietary supplements may be specially formulated for the intended recipients or consumers, such as for adult animals or for older or young animals. For example, a composition adapted for puppies or kittens or adapted for active, pregnant, lactating, or aging animals can be prepared. In general, specialized compositions will comprise energy and nutritional requirements appropriate for animals at different stages of development or age.

Certain aspects of the invention are preferably used in combination with a complete and balanced food. According to certain embodiments provided herein, the compositions comprising the LCPUFA, NORC, and MCTs are preferably used with a high-quality commercial food. As used herein, "high-quality commercial food" refers to a diet manufactured to produce the digestibility of the key nutrients of 80% or more, as set forth in, for example, the recommendations of the National Research Council above for dogs, or in the guidelines set forth by the Association of American Feed Control Officials. Similar high nutrient standards would be used for other animals.

In one embodiment, the food compositions comprise any of a variety of ingredients or combinations thereof selected for their contributions to the overall composition. Thus a skilled food technologist may choose from among natural (*e.g.*, plant or plant-derived, animal or animal-derived, and microbial or microbially-derived), and synthetic ingredients or components. In particular embodiments, the ingredients may include any of the cereal grains and/or fractions or components thereof, meat and meat by-products, fish, shellfish, or other seafood, other animal products or by-products, eggs from any source, vitamins, minerals, salts, sweeteners, fiber, flavoring or other palatants, coloring, and functional ingredients such as emulsifiers, stabilizers, softeners, functional coatings, and the like. Cereals useful in the invention include all plants recognized as "cereal" crops, whether currently used in commercial agriculture or merely known practically or botanically as being a "cereal". For example, "cereals" includes corn, wheat, rice, barley, sorghum, millet, oats, rye, triticale, buckwheat, fonio, and quinoa. The skilled artisan will appreciate that in a given food composition, it is not uncommon to use one or more such cereal products. Meats useful in the invention include products from any animal, preferably muscle tissue such as chicken or other poultry, lamb, sheep, veal, beef, or pork. Other animal products and by-products useful in the invention include dairy products or by-products derived from the milk of any species. Other important components or ingredients include fats and the skilled artisan will appreciate that many sources of vegetable, animal, or microbial fats are available for formulating food compositions. In one embodiment, the source of fat is a plant fat such as corn, soy, or canola oil, preferably one that is readily available. In another embodiment, an animal fat, such as tallow, is useful for providing calories from fat, as well as enticing flavor to meat-eating animals. Of course, combinations of any of the foregoing ingredients, such as fats, are known in the art and useful for optimizing the food compositions based on functional properties as well as price and availability.

The skilled artisan will also appreciate that in formulating the food compositions of the invention, the formulation may vary slightly, so as to allow consideration by the formulator of the price and/or availability of certain ingredients in the compositions, as well as the batch-to-batch variation in the analysis of certain ingredients. Thus a given food composition or formulation may vary slightly from batch to batch, plant to plant, or even season to season depending on such factors. Notwithstanding such variation in specific ingredients selected for manufacturing a particular batch of a food composition, the overall composition (for example, analysis of protein, carbohydrate, fat, fiber, or other component) may be held constant or at least substantially constant, for example, in accordance with a label claim, such as a claim or guarantee of a minimum or maximum percent of a particular component.

In other embodiments, the compositions of the invention comprise LCPUFA, NORC, and MCTs, and, if included in the composition, B vitamins and antioxidants in an amount effective for one or more of reducing or preventing a decline of social interaction, reducing or preventing age-related behavioral changes, increasing trainability, maintaining optimal brain function, facilitating learning and memory, reducing memory loss, retarding brain aging, preventing or treating strokes, and preventing or treating dementia in an animal.

The skilled artisan will understand how to determine the appropriate amount of LCPUFA, NORC, and MCTs, B vitamins, antioxidants, and any other ingredients to be added to a given composition. The skilled formulator may consider important the animal's species, age, size, weight, health, and the like in determining how best to formulate a particular composition, food, or pharmaceutical composition comprising the LCPUFA, NORC, and MCTs and other components. Other factors that may be taken into account for formulation include the type of composition (*e.g*., pet food composition versus dietary supplement), the desired dosage of each component (LCPUFA, NORC, and MCTs), the average consumption of specific types of compositions by different animals (*e.g*., based on species, body weight, activity/energy demands, and the like) and the manufacturing conditions under which the composition is prepared. Preferably, the concentrations of LCPUFA, NORC, and MCTs to be added to the composition are calculated on the basis of the energy and nutrient requirements of the animal. When formulating the compositions of the present invention, a skilled can determine the amounts of the LCPUFA, NORC, and MCTs and other components of the compositions and of other compounds or ingredients, in for example a food composition, based upon the desired dosages and the characteristics of the animal.

For pet foods and food products formulated for human consumption, the amount of LCPUFA as a percentage of the composition is preferably in the range of about 0.1% to about 13% of the composition, although a greater percentage can be supplied. In various embodiments, the amount of LCPUFA is about 0.1%, 0.2%, 0,3%, 0.4%, 0.5%, 0.6%, 07% 0.8%, 0.9%, 1.0%, 1.1%, 1.2%, 1.3%, 1.4%, 1.5%, 1.6%, 1.7%, 1.8%, 1.9%; 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%; 4.9%, 5.0%, or more, *e.g*., 6%, 7%, 8%, 9%, 10%, 11%, 12%, 13%, 14%, 15%, 16%, 17%, 18%, 19%, 20% or more, of the composition. Up to 30% LCPUFA may be used in certain embodiments.

For pet foods and food products formulated for human consumption, the amount of MCTs as a percentage of the composition is in the range of about 1% to about 20% of the composition, although a lesser or greater percentage can be supplied. In various embodiments, the amount is about 1.0%, 1.5%, 2.0%, 2.5%, 3.0%, 3.5%, 4.0%, 4.5%, 5.0%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5%, 20%, of the composition. Dietary supplements may be formulated to contain several fold higher concentrations of MCTs, to be amenable for administration to an animal in the form of a tablet, capsule, liquid concentrate, or other similar dosage form, or to be diluted before administration, such as by dilution in water, spraying or sprinkling onto a pet food, and other similar modes of administration.

For pet foods and food products formulated for human consumption, the amount of NORC as a percentage of the composition is in the range of about 1% to about 10% of the composition, although a greater percentage can be supplied. In various embodiments, the amount of NORC is about 1.0%, 1.1%, 1.2%, 1.3%, 14%, 1.5%, 1.6%, 1.7%, 1.8%, 1.9%, 2.0%, 2.1%, 2.2%, 2.3%, 2.4%, 2.5%, 2.6%, 2.7%, 2.8%, 2.9%, 3.0%, 3.1%, 3.2%, 3.3%, 3.4%, 3.5%, 3.6%, 3.7%, 3.8%, 3.9%, 4.0%, 4.1%, 4.2%, 4.3%, 4.4%, 4.5%, 4.6%, 4.7%, 4.8%, 4.9%, 5.0%, or more, *e.g*., 6%, 7%, 8%, 9%, 10% of the composition. In specific embodiments, 2-2.5% LCPUFA and 2-2.5% NORC are used. In such embodiments, pure L-arginine is a preferred NORC compound. Dietary supplements may be formulated to contain several-fold higher concentrations of LCPUFA and NORC, to be amenable for administration to an animal in the form of a tablet, capsule, liquid concentrate, emulsion, suspension, gel, or other dosage form, or to be diluted before administration, such as by dilution in water, or adding to a pet food (for example, by spraying or sprinkling thereon), and other modes of administration suitable for administering such dietary supplements

In an alternative embodiment, the amount of LCPUFA and NORC in the composition is a function of an amount required to establish specified concentrations, or a desired range of concentrations, of LCPUFA and/or NORC, or a metabolite thereof, in the blood serum of the animal. The specified concentrations, or desired ranges of LCPUFA and/or NORC in the blood serum may be calculated by determining the blood serum levels of animals fed the recommended amounts of LCPUFA and NORC specified above, as would be appreciated by one of skill in the art.

In a preferred embodiment, the food compositions comprise a macronutrient composition suitable for the type of food being designed. In one embodiment, the food composition has about 20 to 32% protein, about 30 to 50% carbohydrate, about 5% to 20% fat, and about 15% to 25% moisture. In another embodiment, the food composition is a pet food composition such as a premium or super-premium pet food composition. In one embodiment, the pet food is formulated for canines and has a protein content of about 20-30%, preferably about 24-28%, and more preferably about 25-27%. In one embodiment, the protein content of a dog food composition is about 26% by weight. In another embodiment, the formulation is for felines and has a protein content of about 35-45%, preferably about 37-42%, and more preferably about 39-41%. In one embodiment, the protein content of a cat food composition is about 40%. In a preferred embodiment, the composition is a food product comprising LCPUFA, NORC, and MCTs, and further comprising about 15% to about 50% protein, about 5% to about 40% fat, about 5% to about 10% ash content, and having a moisture content of about 5% to about 20%.

In one embodiment, the food composition is a wet food, such as a canned food, frozen food, or fresh food product. In one embodiment, the food composition is shelf stable. In another, it must be refrigerated. In other embodiments, the food composition is an intermediate moisture product or a dry food product as described above

In a preferred embodiment, the LCPUFA is a fish oil and the NORC is arginine or a nitric oxide-releasing derivative thereof. In certain embodiments, the compositions comprise from about 0.1% to about 50% fish oil and from about 0.1% to about 20% arginine. In preferred embodiments, both LCPUFA and NORC are within the preferred ranges provided herein when the composition is a food product. Such ranges offer improved palatability and functional properties that will enhance acceptance of the food product. Preferably MCTs are in the preferred range as well. Excessive MCTs may have a tendency to soak or stain certain packaging material that may absorb the oil, thus, proper choices should be made for packaging the food composition.

In various embodiments, the composition is a human food composition, pet food composition, or a dietary supplement. The composition that is a dietary supplement may contain vastly different concentrations or amounts of the LCPUFA, NORC, and MCTs than a food product or pet food. Generally, the palatability and similar sensory factors are not of concern with certain dietary supplements, *e.g.*, those that are swallowed.

In one embodiment, the LCPUFA comprises one or more of a natural fish oil, ALA, EPA, DPA, DHA, or another n-3 fatty acid from any source. Combinations of LCPUFA sources are of course contemplated for use herein. LCPUFA with n-3 or n-6 are also contemplated for use in various embodiments.

Preferably, the composition is formulated for a companion animal, *e.g.*, a dog or cat. In other embodiments, the animal is a human, with or without an age-related cognitive decline.

In one embodiment, the composition is formulated to provide about 0.5g to about 10g each of LCPUFA and NORC per day in one or more portions of a recommended serving size. For example, if the composition is intended for twice-per-day consumption, a 10g dose can be provided by formulating the composition such that each recommended portion (*e.g.*, 2 spoonsful, or one 20g portion, or the like) to provide 5g each of LCPUFA and NORC.

In a preferred embodiment, the compositions comprise from about 0.1% to about 30% medium chain triglycerides of Formula I wherein the R', R", and R'" esterified to the glycerol backbone are each independent fatty acids having 5-12 carbons; from about 0.1% to about 50% fish oil; and from about 0:1% to about 20% arginine. Preferably, the compositions further comprise from about 0.1 to 40 times the recommended daily requirement of B vitamins, from about 0.1 to 25 times the recommended daily requirement of antioxidants, or both. In one embodiment, the composition used in the methods comprises from about 10g to about 30g of MCT, from about 0.5g to about 10g LCPUFA, and from about 0.5g to about 10g NORC, with or without the RDA for B vitamins and antioxidants.

In another aspect, the compositions further comprise one or more cognitive drugs in an amount effective for enhancing cognitive function. The skilled artisan can determine the amount of cognitive drug to be added to the composition based upon the recommended dosage for the drug given by its manufacturer or upon the animal's weight, species, age, health status, and the like.

In another aspect, the disclosure describes pharmaceutical compositions comprising a composition of the present invention and one or more pharmaceutically-acceptable carriers, diluents, or excipients. Generally, pharmaceutical compositions are prepared by admixing a compound or composition with excipients, buffers, binders, plasticizers, colorants, diluents, compressing agents, lubricants, flavorants, moistening agents, and the like, including other ingredients known to skilled artisans to be useful for producing pharmaceuticals and formulating compositions that are suitable for administration to an animal as pharmaceuticals. Optionally, the pharmaceutical compositions further comprise one or both of a B vitamin and an antioxidant.

In another aspect, the invention provides uses for enhancing cognitive function in an animal. The uses generally comprise administering to an animal a composition comprising one or more long chain polyunsaturated fatty acids (LCPUFA), one or more medium chain triglycerides (MCTs), and one or more nitric oxide releasing compounds (NORC) to the animal in an amount effective for enhancing cognitive function in the animal. The compositions further comprise one or more B vitamins, one or more antioxidants, or combinations thereof.

In some embodiments, the LCPUFA, MCTs, NORCs, B vitamins, and antioxidants are administered to the animal in amounts given herein when describing the compositions. In one embodiment, the composition is administered in an amount effective to increase one or more cognitive, motor, or behavioral functions in animals, including animals of all ages that are healthy or animals that are susceptible to or suffering from a decline in cognitive function brought about by the aging process or by disease. In another embodiment, the composition is administered in an amount effective to prevent, reduce, or delay a decline in cognitive function in animals, particularly aging animals susceptible to or suffering from a decline in cognitive function brought about by the aging process or by disease. In certain embodiments, the daily dose for the compositions ranges from about 5 mg/day to about 5,000 mg/day, 10,000 mg/day, or 20,000, or more per animal. Preferably, the daily dose ranges from about 30 mg/day to about 10,000 mg/day per animal, and more preferably from about 750 mg/day to about 7,500 mg/day per animal. The daily dose of LCPUFA, NORC, and MCTs can be measured in terms of grams of LCPUFA, NORC, and MCTs per kg of body weight (BW) of the animal. The daily dose of LCPUFA, NORC, and MCTs thereof can range from about 0.001 g/kg to about 50 g/kg BW of the animal, although greater or lesser doses can be provided. Preferably, the daily dose of LCPUFA, NORC, and MCTs is from about 0.001g/kg to about 25 g/kg BW of the animal. More preferably, the daily dose of LCPUFA, NORC, and MCTs thereof is from about 0.001 g/kg to about 10 g/kg BW of the animal. More preferably, the daily dose of LCPUFA, NORC, and MCTs is from about 0.001 g/kg to about 5 g/kg BW of the animal. More preferably, the daily dose of LCPUFA, NORC, and MCTs is from about 0.001 g/kg to about 1 g/kg BW of the animal. More preferably, the daily dose of the LCPUFA, NORC, and MCTs is from about 0.001 g/kg to about 0.5 g/kg BW of the animal.

Administration in accordance with the uses can be on an as-needed or as-desired basis of varying or regular frequency. A goal of regular ingestion is to provide the animal with a regular and consistent dose of the composition or the direct or indirect metabolites that result from such ingestion. Such regular and consistent dosing will tend to create constant blood levels of the components of the compositions or their direct or indirect metabolites. Thus, regular administration can be once monthly, once weekly, once daily, or more than once daily. Similarly, administration can be every other day, week, or month, every third day, week, or month, every fourth day, week, or month, and the like. Administration can be multiple times per day; When utilized as a supplement to ordinary dietetic requirements, the composition may be administered directly to the animal, *e.g.*, orally, or otherwise. The compositions can alternatively be contacted with, or admixed with, daily feed or food, including a fluid, such as drinking water, or an intravenous connection for an animal that is receiving such treatment. When utilized as a daily feed or food, administration will be well known to those of ordinary skill.

Administration can also be carried out as part of a dietary regimen for the animal. For example, a dietary regimen may comprise causing the regular ingestion by the animal of a composition described herein in an amount effective to prevent, reduce, or delay a decline in one or more cognitive, motor, or behavioral functions in the animal.

According to the uses of the invention, administration of the compositions, including administration as part of a dietary regimen, can span a period of time ranging from parturition through the adult life of the animal. In various embodiments, the animal is a human or companion animal such as a dog or cat. In certain embodiments, the animal is a young or growing animal, In more preferred embodiments, the animal is an aging animal. An animal that has reached about 35% of its projected lifespan is generally preferred. In presently preferred embodiments administration begins, for example, on a regular or extended regular basis, when the animal has reached more than about 30%, 40%, or 50% of its projected or anticipated lifespan. In some embodiments, the animal has attained 40, 45, or 50% of its anticipated lifespan. In yet other embodiments, the animal is older having reached 60, 66, 70, 75, or 80% of its likely lifespan. A determination of lifespan may be based on actuarial tables, calculations, estimates, of the like, and may consider past, present, and future influences or factors that are known to positively or negatively affect lifespan. Consideration of species, gender, size, genetic factors, environmental factors and stressors, present and past health status, past and present nutritional status, stressors, and the like may also influence or be taken into consideration when determining lifespan.

In various embodiments of the uses, the composition is a human food composition, pet food composition, or a dietary supplement. In other embodiments, the composition is a food composition further comprising about 15% to about 50% protein, about 5% to about 40% fat, about 5% to about 10% ash content, and having a moisture content of about 5% to about 20%.

Preferably, the composition being administered comprises from about 1% to about 30% MCTs. Moreover, preferably the MCTs are structured such that greater than about 95% of the R', R", and R"' are 8 carbons in length. The remaining R', R", and R'" are 6-carbon or 10-carbon fatty acids in certain embodiments.

In certain embodiments, the LCPUFA is a fish oil and the NORC is arginine or a nitric oxide-releasing derivative thereof. The MCTs are preferentially sourced from coconut oil, though other sources may be used.

In certain embodiments, the composition being administered comprises from about 0.1% to about 50% fish oil and from about 0.1% to about 20% arginine. For embodiments of the methods, as with the compositions above, the LCPUFA comprises one or more of a natural fish oil, ALA, EPA, DPA, DHA, another n-3 fatty acid from any source, or combinations thereof.

In one embodiment, the composition used in the methods is formulated to provide about 0.5 g to about 10 g each of LCPUFA and NORC per day via one or more portions of a recommended serving size. The composition can further comprise one or more B vitamins and one or more antioxidants. The B vitamin comprises from about 0.1 to about 40 times the RDA and the antioxidant from about 0.1% to about 25% of the composition. Preferably, the composition comprises a mixture of B vitamins.

In one embodiment, the composition is administered to the animal in conjunction with one or more cognitive drugs in an amount effective for enhancing cognitive and related functions as defined herein. In a particular embodiment, the composition administered is the pharmaceutical composition that includes a cognitive drug along with LCPUFA, NORC, and MCTs In a preferred embodiment, the composition is administered to the animal on a daily basis, preferably in a single dose.

In certain embodiments, the animal is a healthy aging animal. In others, the animal has a phenotype associated with age-related cognitive impairment. For example, when compared to a control animal not having the phenotype, the animal may have a phenotype that includes one or more of decreased ability to recall, short-term memory loss, decreased learning rate, decreased capacity for learning, decreased problem solving skills, decreased attention span, decreased motor performance, increased confusion, or dementia (Alzheimer's in humans or its equivalent in other animals).

In another aspect, the composition comprises LCPUFA, NORC, and MCTs in an amount effective for improving one or more social behaviors, In a preferred embodiment, the animal is a companion animal.

The compositions, after being administered to an animal, are believed to increase the circulating concentrations of LCPUFA, NORC, and MCTs in the blood or blood plasma of the animal. In certain embodiments, the blood concentration of a direct or indirect metabolite that results from the consumption of LCPUFA, NORC, and/or MCTs is increased, and is useful as an indicator of dose. A decrease in concentration of one or more compounds in the bloodstream of an animal receiving the compositions can also occur in the normal course. Such decrease can be also be a useful tool for monitoring or determining dosages. Preferably, the change in amount of the bloodstream component to be measured is dosage dependent. In some embodiments, the method results in an increase in one or more ketone bodies in the animal's blood. In embodiments where additional compounds are included in the compositions, *e.g.*, B vitamins or antioxidants, administration results in an increase in circulating concentrations of these compounds and is useful as an indication of dose.

The invention also provides other uses for affecting various cognitive, motor, or behavioral related functions and for affecting various physiological functions that relate to cognitive, motor, or behavioral functions, *e.g.,* social interaction and brain aging. Generally, the uses comprise administering the compositions of the present invention to an animal in amounts defined herein for the compositions and methods.

In one aspect, the invention provides uses for reducing or preventing a decline of social interaction in an animal comprising administering a composition comprising LCPUFA, NORC, and MCTs to the animal in an amount effective to reduce or prevent a decline in social interaction. In preferred embodiments, the compositions further comprise one or more B vitamins, one or more antioxidants, or both in amounts effective for reducing or preventing a decline of social interaction in an animal.

In another aspect, the invention provides uses for reducing or preventing age-related behavioral changes in an animal comprising administering LCPUFA, NORC, and MCTs to the animal in an amount effective for reducing or preventing age-retated behavioral changes in the animal. Preferably, the age-related behavioral changes are one or more of forgetfulness, disorientation, reduced social interaction, changes in sleep and wake habits, loss of "housetraining", confusion, frustration, or change in temperament. Such changes have been noted in response to cognitive declines in humans and other animals. In preferred embodiments, the compositions further comprise one or more B vitamins, one or more antioxidants, or both in amounts effective for reducing or preventing age-related behavioral changes in an animal.

In another aspect, the invention provides uses for increasing trainability of an animal comprising administering a composition comprising LCPUFA, NORC, and MCTs to the animal in an amount effective for increasing trainability. For example, administering the composition while "potty training" babies, puppies, and kittens permits the animal to learn the task more quickly than if the training occurred without using the composition. Similarly, training a dog or cat to obey verbal, signal, or other commands permits the animal to learn the task more quickly than if the training occurred without using the composition. Similarly, the compositions could be useful for training feral or wild animals such as animals used in a circus or pets raised in the wild. In preferred embodiments, the compositions further comprise one or more B vitamins, one or more antioxidants, or both in amounts effective for increasing trainability of an animal.

In another aspect, the invention provides uses for maintaining optimal brain function in an animal. The uses comprise administering a composition comprising LCPUFA, NORC, and MCTs to the animal in an amount effective to prevent or delay a decline in brain function, particularly over time. In preferred embodiments, the compositions further comprise one or more B vitamins, one or more antioxidants, or both in amounts effective for maintaining optimal brain function in an animal.

In another aspect, the invention provides uses for facilitating learning and memory in an animal. The uses comprise administering a composition comprising LCPUFA, NORC, and MCTs to the animal in an amount effective for facilitating learning and memory in the animal. In one embodiment, the animal has reached at least about 50% of its life expectancy. In preferred embodiments, the compositions further comprise one or more B vitamins, one or more antioxidants, or both in amounts effective for facilitating learning and memory in an animal.

In another aspect, the invention provides uses for reducing memory loss in an animal. The uses comprise administering a composition comprising LCPUFA, NORC, and MCTs to the animal in an amount effective to reduce memory loss over time. In preferred embodiments, the compositions further comprise one or more B vitamins, one or more antioxidants, or both in amounts effective for reducing memory loss in an animal.

In another aspect, the invention provides uses for retarding brain aging in an animal. The uses comprise administering a composition comprising LCPUFA, NORC, and MCTs to the animal in an amount effective to retard brain aging. In preferred embodiments, the compositions further comprise one or more B vitamins, one or more antioxidants, or both in amounts effective for retarding brain aging in an animal.

In another aspect, the invention provides uses for preventing or treating strokes in an animal. The uses comprise administering a composition comprising LCPUFA, NORC, and MCTs to the animal in an amount effective to prevent or treat strokes. In preferred embodiments, the compositions further comprise one or more B vitamins, one or more antioxidants, or both in amounts effective for preventing or treating strokes in an animal. The uses are based upon the discovery that reducing damage that results from strokes is correlated to certain aspects of enhancing cognitive function, *e.g*., reducing memory loss.

In another aspect, the invention provides uses for preventing or treating dementia in an animal. The uses comprise administering a composition comprising LCPUFA, NORC, and MCTs to the animal in an amount effective to prevent or treat dementia. In preferred embodiments, the compositions further comprise one or more B vitamins, one or more antioxidants, or both in amounts effective for preventing or treating dementia in an animal. Dementia can be Alzheimer's Disease (AD) in humans, Canine Cognitive Dysfunction Syndrome (CCDS) in canines, or similar diseases in other animals. The uses are based upon the discovery that the compositions and methods of the present invention prevent or reduce dementia by reducing the effects of damage that results from the causes of dementia, *e.g*., amyloid deposits or deterioration of artery function.

In the uses for reducing or preventing a decline of social interaction, reducing or preventing age-related behavioral changes, increasing trainability, maintaining optimal brain function, facilitating learning and memory, reducing memory loss, retarding brain aging, preventing or treating strokes, and preventing or treating dementia, the animal is preferably a human or companion animal, most preferably a dog or cat. The amounts of LCPUFAs, NORCs, MCTs, B vitamins, antioxidants, and other ingredients used in these uses are the same as the amounts or within the ranges given for enhancing cognitive function.

In a further aspect, the invention provides kits as defined in the claims suitable for administering a composition comprising one or more LCPUFAs, NORCs and MCTs to an animal. The kits comprise in separate containers in a single package or in separate containers in a virtual package, as appropriate for the kit component, (a) one or more LCPUFAs, (b) one or more NORCs, (c) one or more MCTs and one or more of (1) one or more other ingredients suitable for consumption by an animal; (2) one or more B vitamins; (3) one or more antioxidants; (4) one or more cognitive drugs; (5) one or more prebiotics; (6) one or more probiotics; (7) one or more diagnostic devices suitable for determining whether an animal could benefit from compositions and methods for enhancing cognitive function and related functions; (8) instructions for how to combine or prepare the LCPUFA, NORC, and MCTs and any other ingredients provided in the kit for administration to an animal; (9) instructions for how to use the combined kit components, prepared kit components, or other kit components for the benefit of an animal; and (10) a device for administering the combined or prepared kit components to an animal. The components are each provided in separate containers in a single package or in mixtures of various components in different packages. In preferred embodiments, the kits comprise the LCPUFAs, NORCs, MCTs, B vitamins, and antioxidants. The kits may comprise the ingredients in various combinations. For example, the kit could comprise a mixture of one or more B vitamins and one or more antioxidants in one container and one or more other ingredients in one or more other containers. Similarly, the kit could comprise a mixture of LCPUFA and MCTs in one container and one or more other ingredients in one or more other containers. Other such combinations can be produced by the skilled artisan based upon the characteristics of the ingredients and their physical and chemical properties and compatibilities.

In another aspect, the disclosure describes a means for communicating information about or instructions for one or more of (1) using compositions of the present invention for enhancing cognitive function; (2) admixing the LCPUFAs, NORCs, MCTs, B vitamins, antioxidants, or other components of the invention to produce a composition suitable for enhancing cognitive function; (3) using the kits of the present invention for enhancing cognitive function; and (4) administering the compositions to an animal. The means comprises one or more of a physical or electronic document, digital storage media, optical storage media, audio presentation, audiovisual display, or visual display containing the information or instructions. Preferably, the means is selected from the group consisting of a displayed website, a visual display kiosk, a brochure, a product label, a package insert, an advertisement, a handout, a public announcement, an audiotape, a videotape, a DVD, a CD-ROM, a computer readable chip, a computer readable card, a computer readable disk, a USB device, a FireWire device, a computer memory, and any combination thereof.

In another aspect, the invention provides methods for manufacturing a food composition comprising LCPUFA, NORC, MCTs, and one or more other ingredients suitable for consumption by an animal, *e.g*., protein, fat, carbohydrate, fiber, B vitamins, and antioxidants. The methods comprise admixing one or more ingredients suitable for consumption by an animal with LCPUFA, NORC, and MCTs, and possibly other ingredients such as B vitamins and/or antioxidants. Alternatively, the methods comprise applying LCPUFA, NORC, and MCTs, and other ingredients if desired, separately or in any combination onto the food composition, *e.g*., as a coating or topping. The LCPUFA, NORC, and MCTs can be added at any time during the manufacture and/or processing of the food composition. This includes, for example, admixing the LCPUFA, NORC, and MCTs as part of the core formulation of the "body" of the food composition or applying them as a coating, i.e., primarily to the surface of the food composition after its manufacture. The compositions can be made according to any method suitable in the art.

In another aspect, the present invention provides a package comprising a composition of the present invention and a label affixed to the package containing a word or words, picture, design, acronym, slogan, phrase, or other device, or combination thereof, that indicates that the contents of the package contains a composition suitable for enhancing cognitive function, reducing or preventing a decline of social interaction, reducing or preventing age-retated behavioral changes, increasing trainability, maintaining optimal brain function, facilitating learning and memory, reducing memory loss, retarding brain aging, preventing or treating strokes, or preventing or treating dementia in an animal, particularly an aging animal. Typically, such device comprises the words "improves cognitive function", "improves memory", "reduces memory loss in aging animals", or an equivalent expression printed on the package. Any package or packaging material suitable for containing the composition is useful in the invention, *e.g.*, a bag, box, bottle, can, pouch, and the like manufactured from paper, plastic, foil, metal, and the like. In a preferred embodiment, the package contains a food composition adapted for a particular animal such as a human, canine or feline, as appropriate for the label, preferably a companion animal food composition.

In another aspect, the invention provides for use of LCPUFA, NORC, and MCTs to prepare a medicament for enhancing cognitive function, reducing or preventing a decline of social interaction, reducing or preventing age-related behavioral changes, increasing trainability, maintaining optimal brain function, facilitating learning and memory, reducing memory loss, retarding brain aging, preventing or treating strokes, and preventing or treating dementia in an animal. The medicament can further comprise one or more B vitamins, antioxidants, or combinations thereof. Generally, medicaments are prepared by admixing a compound or composition with excipients, buffers, binders, plasticizers, colorants, diluents, compressing agents, lubricants, flavorants, moistening agents, and other ingredients known to skilled artisans to be useful for producing medicaments and formulating medicaments that are suitable for administration to an animal.

In the invention, the animal can be a juvenile, adult, senior, or geriatric animal. Typically, for most embodiments, the animal is an aging animal. Generally, animals are senior in the last half of their expected lifespan and geriatric in the last fourth of their expected lifespan. Lifespan definitions vary for various animals and is known to skilled artisans. For example, an animal is considered to be juvenile until about 16 years of age. For a dog or cat, the animal is considered to be juvenile until 1 year of age.

The compositions of the invention, including the pharmaceutical compositions and medicaments, are administered to the animal using a variety of administration routes. Such routes include oral, intranasal, intravenous, intramuscular, intragastric, transpyloric, subcutaneous, rectal, and the like. Preferably, the compositions are administered orally.

The compositions of the invention, including the pharmaceutical compositions and medicaments, are administered to the animal for a time required to accomplish one or more objectives of the invention, e.g., enhancing cognitive function, reducing or preventing a decline of social interaction, reducing or preventing age-related behavioral changes, increasing trainability, maintaining optimal brain function, facilitating learning and memory, reducing memory loss, retarding brain aging, preventing or treating strokes, and preventing or treating dementia in an animal The compositions are suitable for long-term administration or administration on any schedule compatible with the composition and objective.

### EXAMPLES

The invention can be further illustrated by the following example, although it will be understood that this example is included merely for purposes of illustration and is not intended to limit the scope of the invention unless otherwise specifically indicated.

### Example 1

### Animal and Test Groups

Forty-eight (48) cognitively-experienced beagle dogs (23 males and 25 females ranging from 7.5 to 11.6 years of age) were tested. The average age of the dogs was 9.38 years. To qualify, the dogs must have had at least 6 months of previous cognitive test experience, including having been trained on the delayed non-matching-to position task (DNMP) and the oddity task.

During an initial baseline phase, all dogs were tested on a variable delay version of the DNMP task (or at 5 s if previously unable to learn the task) that provided a means of assessing visuo-spatial working memory and on a size discrimination and reversal task. Performance on these tasks was used to place the dogs into four cognitively equivalent treatment groups: (1) 5.5% MCT Group; (2) 12.5% MCT Group; (3) 12.5% MCT combined with 2% fish oil, 25% arginine, 1 to 40 times RDA B vitamins, 150 mg/kg vitamin C, 900 mg/kg vitamin E, and 0.5 mg/kg selenium ("Combined Trt Group"); and (4) the Control Group.

### Feeding and Water

Dogs had free access to water via wall-mounted automatic watering systems and/or water bowls. The animals were provided one of five adult maintenance foods having approximatety 32% protein, 20% fat, and 3% fiber once daily. Initial individual food amounts were calculated using the formula "kcal requirement = 110 kcal/day x (BW x 0.75)", where BW is body weight. The formula was intended to maintain a constant body weight at an appropriate body condition.

Dogs were weighed weekly at the start of treatment and twice monthly after food intake and body condition stabilization; food intake was adjusted as needed to maintain a relatively constant body weight. Animals were given approximately 30 minutes to eat the food provided. The approximate amount of food remaining was recorded in a food intake monitoring datasheet for each animal.

### Housing Conditions

### Cognitive Test Battery

Landmark discrimination learning (Days 7-99). Egocentric learning and reversal (Days 100-163).

### Landmark Discrimination Task

In this task, the dogs were trained to approach one of two objects, based on their proximity to an external landmark. The task was intended to assess allocentric spatial ability, which entails utilization of external landmarks to localize objects in space. The dogs were tested on successively more difficult versions of the same general problem.

General test procedures: Dogs were subject to ten (10) trials per day, with an inter-trial interval of 30 seconds. Testing was conducted once per day, approximately six days per week, such that a total of 80 sessions were conducted. A partial correction procedure was used. In this procedure, once each session the dogs are permitted to correct their response after making an error. Each dog was tested on up to four problems, depending upon the dog's success at solving the problems. To move on from the first and second problems, the dog had to complete a two-stage criterion. To pass the first stage, the dog had to respond correctly in at least 9 of 10 trials, or on 8 of 10 trials over 2 consecutive days. Dogs had to respond in all trials to pass the first stage. The second criterion stage was Completed when the dog responded correctly in at least 70% of the next 30 trials over three consecutive sessions. Dogs that had a non-response in any one trial were assigned a score of 0.5, which was assumed to be the response based on random choice, and were given one extra day of testing to complete the 30 trials. An average score of 70% over all test days was required to pass the second stage.

The discriminanda were identical white coasters and a yellow wooden peg, 2 cm x 2 cm x 9 cm, which served as the landmark. The white circular coasters were placed over the two lateral food wells on the presentation tray. Velcro tabs 2 cm in diameter, to hold the landmark in place, were glued to the top center of the coasters and to appropriate loci on the food tray.

Initial Landmark Test (Land-0): On the initial test (L0), the landmark was attached to the center of one of the two white coasters. On each trial, the experimenter placed the food reward in either the left or right food well and positioned the landmark accordingly. The door was raised and the tray was moved to approximately 25 cm from the dog for a brief inspection interval to enable the dog to see the spatial arrangement on the tray. The tray was then presented to the dog and the dog was allowed to respond. In this and all subsequent levels, the dogs were required to respond to the coaster closest to the landmark to obtain the food reward. The correct side was determined randomly by the computer with the constraint that each side was correct on half of the trials of each test session. Each dog was allowed a maximum of 30 test sessions (300 trials) to learn to respond to stimulus associated with the landmark during L0.

Remedial Training: Dogs that failed the initial L0 test were given a remedial training program to help teach them the task. The remedial training consisted of 5 additional training days, with 15 trials per day. At the start of the remedial training, the animals were presented with a single rewarded stimulus on the majority of the trials. With continued testing, additional paired stimulus presentations were given. After completing the remedial learning phase, the animals received additional training, up to a maximum of 10 sessions using the original protocol. This protocol was also used for dogs that failed L1. Dogs that failed L0 were moved on to L1. Dogs that failed remedial training after L1 were moved on to the next task, if 80 test sessions had not elapsed. If fewer that 10 test sessions remained, the animals on L1 received extra remedial training sessions.

Landmark-1 (L1): Once a dog learned the L0 task (either during the initial training or after the remedial training), the landmark was moved 1 cm medially and diagonally away from the edge of the coaster, which constituted landmark 1 (L1). The landmark was attached to the food tray with a black piece of 2 cm wide velcro. Dogs that did not learn within 30 sessions were given remedial training, as described above for L0

### Egocentric Test Protocol.

General Test Procedure: At the start of a trial the hinged door was opened to present the stimulus tray to the dog and the dog was allowed a maximum of 30-sec to make a response by displacing one of the two identical stimuli. A correct response was counted when the dog displaced a stimulus object covering food reward. An error was counted when the dog displaced the non-rewarded stimulus. The animals received 12 trials per session over a period of 63 sessions.

Preference Phase: On the first test day the dogs were given a preference test consisting of 10 discrete trials with objects covering both lateral food wells to determine if any side preferences were present. The preferred side was used as the positive side for the initial acquisition phase of testing. Thus, if the animal chose the object to it's left most frequently, then the animals' left side was designated its preferred side. For animals that did not show a side preference (and responded five times to each side), a coin toss was used to determine the rewarded side.

Acquisition Phase: Each trial consisted of a single presentation of the stimulus tray with a stimulus covering a reward in the preferred side lateral well, or the center well. A second non-rewarded stimulus object covered a well towards the dog's non-preferred side. Consequently, the object furthest to the animals' preferred side was always rewarded. On any given trial, there were three possible spatial configurations (left-center, left, right, or right-center). Each configuration occurred four times per test session.

Criterion: The acquisition phase was successfully completed once a dog scored a minimum of 90% correct (33/36 correct trials) over three consecutive test sessions. A maximum of 12 sessions, or 144 trials, were given to meet this criterion. Animals that did not learn within 12 sessions were given 5 sessions of remedial training, which was followed by up to 10 additional sessions.

Reversal Phase: Once an animal passed the acquisition phase, the rewarded position was switched to the opposite side. Thus, if the object closest to a dog's right was rewarded in acquisition testing, the object closest to its left was rewarded in the reversal testing. For the first reversal test, the learning criterion was the same as for the original acquisition. A maximum of 15 sessions, or 180 trials, were given to meet this criterion. If an animal did not satisfy the criterion within 15 sessions, 5 sessions of remedial training were given. The animals then received 10 additional sessions to reach the learning criterion.

Repeated (Multiple) Reversal Phase: Once an animal learned the original reversal, the correct side was then be switched and the animal retested until it relcarned the task. This procedure was repeated until the animals had completed a total of 52 sessions on the egocentric protocol. The results are shown in the following Tables:

### Landmark Test Results

**Table 1. Landmark-0 Test Results**

| Test Group | Errors to Criterion | |
|---|---|---|
| | Means | SE |
| Control | 45.42 | 12.85 |
| 5.5% MCT | 22.67 | 5.25 |
| 12.5% MCT | 20.42 | 9.14 |
| Combined Trt | 20.33 | 5.62 |

Referring to the data, the Control Group performed worse than other groups.

**Table 2. Landmark-1 Test Results**

| Test Group | Errors to Criterion | |
|---|---|---|
| | Means | SE |
| Control | 136.96 | 16.26 |
| 5.5% MCT | 69.58 | 17.36 |
| 12.5% MCT | 59.46 | 14.56 |
| Combined Trt | 56 | 14.28 |

The Control Group differed significantly from the 5.5% MCT Group, 12.5% MCT Group, and the Combined Trt Group. The Combined Trt Group performed better on both tasks when compared to any of the other groups, demonstrating a synergistic effect.

### Egocentric Test Results

**Table 3. Acquisition Test Results**

| Test Group | Errors to Criterion | |
|---|---|---|
| | Means | SEM |
| Control | 25.58 | 2.84 |
| 5.5% MCT | 15.17 | 2.84 |
| 12.5% MCT | 15.92 | 2.61 |
| Combined Trt | 12.82 | 2.23 |

Referring to the data, the Control Group differed significantly from the 5.5% MCT Group and the Combined Trt Group.

**Table 4. Reversal Test Results**

| Test Group | Errors to Criterion | |
|---|---|---|
| | Means | SEM |
| Control | 68.29 | 5.95 |
| 5.5% MCT | 55.75 | 5.22 |
| 12.5% MCT | 45.00 | 5.80 |
| Combined Trt | 33.91 | 2.41 |

Referring to the data, the Control Group differed significantly from the 12.5% MCT Group and Combined Trt Group. The Combined Trt Group also differed significantly from the 5.5% MCT Group. The Combined Trt Group performed numerically better than any of the other groups, demonstrating an unexpected and synergistic effect.

**Table 5. Repeated Reversal Test Results**

| Test Group | Reversals Learned | |
|---|---|---|
| | Means | SEM |
| Control | 2.42 | 0.40 |
| 5.5% MCT | 4.42 | 0.48 |
| 12 5% MCT | 5.5 | 0.68 |
| Combined Trt | 5.73 | 0.38 |

Referring to the data, the Control Group differed significantly from all other treatment groups. All treatments had beneficial effects. Taken together, the above data show that the compounds administered to the Combined Trt Group (comprising LCPUFA, NORC, and MCTs) effected cognitive function and that there are unexpected effects derived from administering the combination, including some synergistic effects.

In the specification, there have been disclosed typical preferred embodiments of the invention. Although specific terms are employed, they are used in a generic and descriptive sense only and not for purposes of limitation. The scope of the invention is set forth in the claims. Obviously many modifications and variations of the invention are possible in light of the above teachings. It is therefore to be understood that within the scope of the appended claims the invention may be practiced otherwise than as specifically described.

## Claims

1. A composition for use in enhancing cognitive function in an animal wherein cognitive decline is caused by damage from disease or changes in brain function;
the composition comprising from about 0.1 % to about 30 % of one or more long chain polyunsaturated fatty acids (LCPUFA),
from about 1 % to 20 % of one or more medium chain triglycerides (MCTs),
and from about 1 % to 10 % of one or more nitric oxide releasing compounds (NORC) in an amount effective for enhancing cognitive function in an animal;
further comprising one or more B vitamins, and
one or more antioxidants in an amount effective for enhancing cognitive function; comprising from about 0.1 to 40 times the recommended daily requirement of B vitamins and from about 0.1 to 25 times the recommended daily requirement of antioxidants.

2. The composition for use according to claim 1, wherein the disease is strokes, memory loss, dementia; or decline in brain function; Alzheimer's disease, or wherein the animal has a phenotype associated with age-related cognitive impairment.

3. A use of a composition comprising from about 0.1 % to about 30 % of one or more long chain polyunsaturated fatty acids (LCPUFA),
from about 1 % to 20 % of one or more medium chain triglycerides (MCTs), and from about 1 % to 10 % of one or more nitric oxide releasing compounds (NORC) in an amount effective for enhancing cognitive function in an animal;
further comprising one or more B vitamins and
one or more antioxidants in an amount effective for enhancing cognitive function; comprising from about 0.1 to 40 times the recommended daily requirement of B vitamins and from about 0.1 to 25 times the recommended daily requirement of antioxidants;
for the production of a medicament for enhancing cognitive function in an animal wherein cognitive decline is caused by damage from disease or changes in brain function for reducing or preventing decline of social interaction; reducing or preventing age-related behavioral changes.

4. The use according to claim 3, wherein the age-related behavioral changes are one or more of forgetfulness, disorientation, reduced social interaction, changes in sleep and wake habits, loss of "housetraining", confusion, frustration, or change in temperament.

5. The composition for use according to claims 1 or 2, or the use according to any of claims 3 or 4,
(a) wherein the LCPUFA comprises one or more of a natural fish oil, ALA, EPA, DPA, DHA, or another n-3 fatty acid from any source; or
(b) wherein the LCPUFA is a fish oil; or
(c) wherein the MCTs are of a formula (Formula 1): 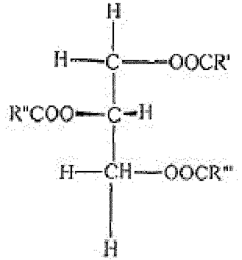 wherein the R', R", and R'" esterified to the glycerol backbone are each independent Fatty acids having 5-12 carbons; optionally wherein greater than about 95% of the R', R", and R'" are 8 carbons in length; optionally wherein the remaining R', R", and R'" are 6-carbon or 10-carbon fatty acids; or
(d) formulated as a human food composition, pet food composition, or a dietary supplement; or
(e) further comprising one or more cognitive drugs in an amount effective for enhancing cognitive function; or
(f) further comprising one or more prebiotics or probiotics.

6. The composition for use according to claim 5 or the use according to claim 5, wherein the NORC is arginine or a nitric oxide-releasing derivative thereof.

7. The composition for use according to claim 5 or the use according to claim 5, wherein the composition is a food composition further comprising about 15% to about 50% protein, about 5% to about 40% fat, about 5% to about 10% ash content, and having a moisture content of about 5% to about 20%.

8. The composition for use according to any of claims 1, 2, 5 to 7 or the use according to any of claims 3 to 7, wherein the composition is a pharmaceutical or nutraceutical composition comprising one or more pharmaceutically or nutraceutically acceptable-carriers, diluents, or excipients.

9. The composition for use according to any of claims 1, 2, 5 to 8 or the use according to any of claims 3 to 8,
(a) wherein the composition is a pharmaceutical or nutraceutical composition that optionally comprises one or more cognitive drugs; or
(b) wherein the animal has a phenotype associated with age-related cognitive impairment; optionally wherein the phenotype includes one or more of decreased ability to recall, short-term memory loss, decreased learning rate, decreased capacity for learning, decreased problem solving skills, decreased attention span, decreased motor performance, increased confusion, or dementia, as compared to a control animal not having the phenotype.

10. The composition for use according to any of claims 1, 2, 5 to 9 or the use according to any of claims 3 to 9, wherein the animal is
(a) a human or companion animal; or
(b) a juvenile or adult animal; or
(c) an aging animal.

11. The composition for use according to any of claims 1, 2, 5 to 9 or the use according any of claims 3 to 9, wherein the animal is an aging animal.

12. A kit suitable for administering a composition comprising from about 0.1 % to 30 % of one or more LCPUFAs, from about 1 % to 10 % of NORCs and from about 1 % to 20 % of MCTs to an animal comprising in separate containers in a single package or in separate containers in a virtual package, as appropriate for the kit component, (a) one or more LCPUFAs, (b) one or more NORCs, (c) one or more MCTs (d) one or more B ingredients suitable for consumption by an animal; (2) one or more cognitive drugs; (3) one or more prebiotics; (4) one or more probiotics; (5) one or more diagnostic devices suitable for determining whether an animal could benefit from compositions and methods for enhancing cognitive function and related functions; (6) instructions for how to combine or prepare the LCPUFA, NORC, and MCTs and any other ingredients provided in the kit for administration to an animal; (7) instructions for how to use the combined kit components, prepared kit components, or other kit components for the benefit of an animal; and (8) a device for administering the combined or prepared kit components to an animal.

13. A method for manufacturing a food composition comprising from about 0.1 % to 30 % of LCPUFA, from about 1 % to 10 % of NORC, from about 1 % to 20 % of MCTs and one or more ingredients suitable for consumption by an animal comprising admixing one or more ingredients suitable for consumption by an animal with LCPUFA, NORC, and MCTs or applying LCPUFA, NORC, and MCTs separately or in any combination onto the food composition; wherein the ingredients suitable for consumption by an animal are one or more B vitamins and one or more antioxidants.

14. A package comprising a composition according to claim 1, and a label affixed to the package containing a word or words, picture, design, acronym, slogan, phrase, or other device, or combination thereof, that indicates that the contents of the package contains a composition suitable for enhancing cognitive function; altering cognitive, motor, or behavioral function; reducing or preventing decline of social interaction; reducing or preventing age-related behavioral changes; increasing trainability; maintaining optimal brain function; facilitating learning and memory; and/or reducing memory loss in an animal.

## Patentansprüche

1. Zusammensetzung zur Verwendung beim Fördern der kognitiven Funktion in einem Lebewesen, wobei der kognitive Verfall durch Schädigung durch eine Erkrankung oder Veränderungen der Gehirnfunktion verursacht wird;
die Zusammensetzung von etwa 0,1 % bis etwa 30 % einer oder mehrerer langkettiger mehrfach ungesättigter Fettsäuren (LCPUFA),
von etwa 1 % bis 20 % einer oder mehrerer mittelkettiger Triglyceride (MCTs),
und von etwa 1 % bis 10 % einer oder mehrerer Stickstoffmonoxid freisetzender Verbindungen (NORC) in einer Menge umfasst, die zum Fördern der kognitiven Funktion in einem Lebewesen wirksam ist;
ferner umfassend eines oder mehrere B-Vitamine, und
eines oder mehrere Antioxidationsmittel in einer Menge, die zum Fördern der kognitiven Funktion wirksam ist; umfassend das etwa 0,1 - bis 40-fache des empfohlenen Tagesbedarfs an B-Vitaminen und das etwa 0,1 - bis 25-fache des empfohlenen Tagesbedarfs an Antioxidationsmitteln.

2. Zusammensetzung zur Verwendung nach Anspruch 1, wobei die Erkrankung Schlaganfälle, Gedächtnisverlust, Demenz; oder Verfall der Gehirnfunktion; Alzheimer-Krankheit ist, oder wobei das Lebewesen einen Phänotyp aufweist, der mit altersbedingter kognitiver Beeinträchtigung in Zusammenhang steht.

3. Verwendung einer Zusammensetzung, umfassend von etwa 0,1 % bis etwa 30 % einer oder mehrerer langkettiger mehrfach ungesättigter Fettsäuren (LCPUFA),
von etwa 1 % bis 20 % einer oder mehrerer mittelkettiger Triglyceride (MCTs) und von etwa 1 % bis 10 % einer oder mehrerer Stickstoffmonoxid freisetzender Verbindungen (NORC) in einer Menge, die zum Fördern der kognitiven Funktion in einem Lebewesen wirksam ist;
ferner umfassend eines oder mehrere B-Vitamine und
eines oder mehrere Antioxidationsmittel in einer Menge, die zum Fördern der kognitiven Funktion wirksam ist; umfassend das etwa 0,1 - bis 40-fache des empfohlenen Tagesbedarfs an B-Vitaminen und das etwa 0,1 - bis 25-fache des empfohlenen Tagesbedarfs an Antioxidationsmitteln;
zur Herstellung eines Medikaments zum Fördern der kognitiven Funktion in einem Lebewesen, wobei der kognitive Verfall durch Schädigung von einer Erkrankung oder Veränderungen der Gehirnfunktion verursacht wird, um den Verfall der sozialen Interaktion zu verringern oder zu verhindern; altersbedingte Verhaltensänderungen zu verringern oder zu verhindern.

4. Verwendung nach Anspruch 3, wobei die altersbedingten Verhaltensänderungen eine oder mehrere von Vergesslichkeit, Desorientierung, verringerter sozialer Interaktion, Änderungen in Schlaf- und Wachgewohnheiten, Verlust von "Haustraining", Verwirrung, Frustration oder Temperamentveränderung sind.

5. Zusammensetzung zur Verwendung nach Anspruch 1 oder 2 oder Verwendung nach einem der Ansprüche 3 oder 4,
(a) wobei die LCPUFA eine oder mehrere von natürlichem Fischöl, ALA, EPA, DPA, DHA oder einer anderen n-3-Fettsäure aus einer beliebigen Quelle umfasst; oder
(b) wobei die LCPUFA ein Fischöl ist; oder
(c) wobei die MCTs eine Formel (Formel 1) aufweisen: 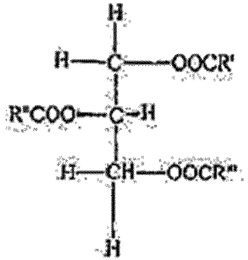 wobei die an das Glyceringrundgerüst veresterten R', R" und R'" jeweils unabhängig Fettsäuren mit 5-12 Kohlenstoffen sind; wahlweise wobei mehr als etwa 95 % der R', R" und R'" 8 Kohlenstoffe lang ist; wahlweise wobei die verbleibenden R', R" und R'" Fettsäuren mit 6 Kohlenstoffatomen oder 10 Kohlenstoffatomen sind; oder
(d) die als Nahrungsmittelzusammensetzung für Menschen, Nahrungsmittelzusammensetzung für Haustiere oder Nahrungsergänzungsmittel formuliert ist; oder
(e) ferner umfassend eines oder mehrere kognitive Arzneimittel in einer Menge, die zum Fördern der kognitiven Funktion wirksam ist; oder
(f) ferner umfassend eines oder mehrere Präbiotika oder Probiotika.

6. Zusammensetzung zur Verwendung nach Anspruch 5 oder Verwendung nach Anspruch 5, wobei NORC Arginin oder ein Stickstoffmonoxid freisetzendes Derivat davon ist.

7. Zusammensetzung zur Verwendung nach Anspruch 5 oder Verwendung nach Anspruch 5, wobei die Zusammensetzung eine Nahrungsmittelzusammensetzung ist, ferner umfassend etwa 15 % bis etwa 50 % Protein, etwa 5 % bis etwa 40 % Fett, etwa 5 % bis etwa 10 % Aschegehalt und mit einem Feuchtigkeitsgehalt von etwa 5 % bis etwa 20 %.

8. Zusammensetzung zur Verwendung nach einem der Ansprüche 1, 2, 5 bis 7 oder Verwendung nach einem der Ansprüche 3 bis 7, wobei die Zusammensetzung eine pharmazeutische oder nutrazeutische Zusammensetzung ist, umfassend einen oder mehrere pharmazeutisch oder nutrazeutisch verträgliche Träger, Verdünnungsmittel oder Hilfsstoffe.

9. Zusammensetzung zur Verwendung nach einem der Ansprüche 1, 2, 5 bis 8 oder Verwendung nach einem der Ansprüche 3 bis 8,
(a) wobei die Zusammensetzung eine pharmazeutische oder nutrazeutische Zusammensetzung ist, die wahlweise eines oder mehrere kognitive Arzneimittel umfasst; oder
(b) wobei das Lebewesen einen Phänotyp aufweist, der mit altersbedingter kognitiver Beeinträchtigung in Zusammenhang steht; wahlweise wobei der Phänotyp eines oder mehrere von verminderter Erinnerungsfähigkeit, Kurzzeitgedächtnisverlust, verminderter Lerngeschwindigkeit, verminderter Lernkapazität, verminderten Problemlösefähigkeiten, verminderter Aufmerksamkeitsspanne, verminderter motorischer Leistung, vermehrter Verwirrung oder Demenz im Vergleich mit einem den Phänotyp nicht aufweisenden Kontrolllebewesen einschließt.

10. Zusammensetzung zur Verwendung nach einem der Ansprüche 1, 2, 5 bis 9 oder Verwendung nach einem der Ansprüche 3 bis 9, wobei das Lebewesen
(a) ein Mensch oder ein Haustier; oder
(b) ein juveniles oder erwachsenes Lebewesen; oder
(c) ein alterndes Lebewesen ist.

11. Zusammensetzung zur Verwendung nach einem der Ansprüche 1, 2, 5 bis 9 oder Verwendung nach einem der Ansprüche 3 bis 9, wobei das Lebewesen ein alterndes Lebewesen ist.

12. Kit, geeignet zum Verabreichen einer Zusammensetzung, umfassend von etwa 0,1 % bis 30 % einer oder mehrerer LCPUFAs, von etwa 1 % bis 10 % NORCs und von etwa 1 % bis 20 % MCTs, an ein Lebewesen, umfassend in separaten Behältern in einer einzelnen Verpackung oder in separaten Behältern in einer virtuellen Verpackung, wie für die Kit-Komponente geeignet, (a) eine oder mehrere LCPUFAs, (b) eine oder mehrere NORCs, (c) eine oder mehrere MCTs, (d) eines oder mehrere B-Vitamine und eines oder mehrere Antioxidationsmittel und einen oder mehrere von (1) einem oder mehreren anderen Inhaltsstoffen, die zum Verzehr durch ein Lebewesen geeignet sind; (2) einem oder mehreren kognitiven Arzneimitteln; (3) einem oder mehreren Präbiotika; (4) einem oder mehreren Probiotika; (5) einem oder mehreren diagnostischen Vorrichtungen, geeignet zum Bestimmen, ob ein Lebewesen von Zusammensetzungen und Verfahren zum Fördern der kognitiven Funktion und verwandter Funktionen profitieren könnte; (6) Anweisungen zum Kombinieren oder Herstellen der LCPUFA, NORC und MCTs und beliebiger anderer Inhaltsstoffe, die in dem Kit zur Verabreichung an ein Lebewesen bereitgestellt werden; (7) Anweisungen zum Verwenden der kombinierten Kitkomponenten, hergestellten Kitkomponenten oder anderer Kitkomponenten für den Nutzen eines Lebewesens; und (8) eine Vorrichtung zum Verabreichen der kombinierten oder hergestellten Kitkomponenten an ein Lebewesen.

13. Verfahren zum Herstellen einer Nahrungsmittelzusammensetzung, umfassend von etwa 0,1 % bis 30 % LCPUFA, von etwa 1 % bis 10 % NORC, von etwa 1 % bis 20 % MCTs und einen oder mehrere Inhaltsstoffe, geeignet zum Verzehr durch ein Lebewesen, umfassend das Mischen eines oder mehrerer Inhaltsstoffe, geeignet zum Verzehr durch ein Lebewesen, mit LCPUFA, NORC und MCTs oder das Anwenden von LCPUFA, NORC und MCTs separat oder in einer beliebigen Kombination auf die Nahrungsmittelzusammensetzung; wobei die zum Verzehr durch ein Lebewesen geeigneten Inhaltsstoffe eines oder mehrere B-Vitamine und eines oder mehrere Antioxidationsmittel sind.

14. Verpackung, umfassend eine Zusammensetzung nach Anspruch 1 und ein Etikett, das an der Verpackung befestigt ist, das ein Wort oder Wörter, ein Bild, ein Design, ein Akronym, einen Slogan, einen Ausdruck oder eine andere Vorrichtung oder eine Kombination davon enthält, die anzeigt, dass der Inhalt der Verpackung eine Zusammensetzung, geeignet zum Fördern der kognitiven Funktion; Ändern der kognitiven, motorischen oder Verhaltensfunktion; Verringern oder Verhindern des Verfalls der sozialen Interaktion; Verringern oder Verhindern altersbedingter Verhaltensänderungen; Erhöhen der Trainierbarkeit; Halten der optimalen Gehirnfunktion; Erleichtern von Lernen und Gedächtnis; und/oder Verringern von Gedächtnisverlust in einem Lebewesen enthält.

## Revendications

1. Composition pour une utilisation dans l'amélioration de la fonction cognitive chez un animal dans laquelle le déclin cognitif est causé par des dommages provoqués par une maladie ou des changements dans la fonction cérébrale ;
la composition comprenant d'environ 0,1 % à environ 30 % d'un ou plusieurs acides gras polyinsaturés à chaîne longue (AGPCL),
d'environ 1 % à 20 % d'un ou plusieurs triglycérides à chaîne moyenne (TCM),
et d'environ 1 % à 10 % d'un ou plusieurs composés libérant de l'oxyde nitrique (CLON) en une quantité efficace pour améliorer la fonction cognitive chez un animal ;
comprenant également une ou plusieurs vitamines B, et
un ou plusieurs antioxydants en une quantité efficace pour améliorer la fonction cognitive ;
comprenant d'environ 0,1 à 40 fois l'apport quotidien recommandé de vitamines B et d'environ 0,1 à 25 fois l'apport quotidien recommandé d'antioxydants.

2. Composition pour une utilisation selon la revendication 1, dans laquelle la maladie est un accident vasculaire cérébrale, une perte de mémoire, la démence ; ou un déclin de la fonction cérébrale ; la maladie d'Alzheimer, ou dans laquelle l'animal présente un phénotype associé à une déficience cognitive liée à l'âge.

3. Utilisation d'une composition comprenant d'environ 0,1 % à environ 30 % d'un ou plusieurs acides gras polyinsaturés à chaîne longue (AGPCL),
d'environ 1 % à 20 % d'un ou plusieurs triglycérides à chaîne moyenne (TCM), et d'environ 1 % à 10 % d'un ou plusieurs composés libérant de l'oxyde nitrique (CLON) en une quantité efficace pour améliorer la fonction cognitive chez un animal ;
comprenant également une ou plusieurs vitamines B et
un ou plusieurs antioxydants en une quantité efficace pour améliorer la fonction cognitive ;
comprenant d'environ 0,1 à 40 fois l'apport quotidien recommandé de vitamines B et d'environ 0,1 à 25 fois l'apport quotidien recommandé d'antioxydants ;
pour la préparation d'un médicament pour améliorer la fonction cognitive chez un animal dans laquelle le déclin cognitif est entraîné par des lésions provoquées par une maladie ou des changements de la fonction cérébrale pour réduire ou prévenir le déclin de l'interaction sociale ; la réduction ou la prévention des changements comportementaux liés à l'âge.

4. Utilisation selon la revendication 3, dans laquelle les changements comportementaux liés à l'âge sont l'un ou plusieurs parmi oubli, désorientation, interaction sociale réduite, changements dans les habitudes de sommeil ou de réveil, perte de « l'apprentissage de la propreté », confusion, frustration, ou changement de tempérament.

5. Composition pour une utilisation selon les revendications 1 ou 2, ou utilisation selon l'une quelconque des revendications 3 ou 4,
(a) dans laquelle l'AGPCL comprend un ou plusieurs parmi une huile naturelle de poisson, ALA, EPA, DPA, DHA, ou un autre acide gras n-3 provenant de n'importe quelle source ; ou
(b) dans laquelle l'AGPCL est une huile de poisson ; ou
(c) dans laquelle les TCM sont d'une formule (Formule 1) : 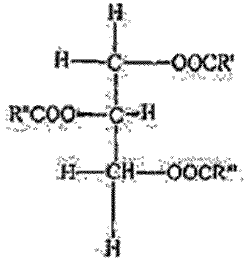 dans laquelle les R', R", et R'" estérifiés au squelette glycérol sont chacun des acides gras indépendants ayant 5 à 12 carbones ; éventuellement dans laquelle plus d'environ 95 % des R', R", et R'" ont une longueur de 8 carbones ; éventuellement dans laquelle les R', R", et R'" restants sont des acides gras à 6 carbones ou 10 carbones ; ou
(d) formulée en tant composition alimentaire humaine, composition alimentaire pour animaux de compagnie, ou complément alimentaire ; ou
(e) comprenant également un ou plusieurs médicaments cognitifs en une quantité efficace pour améliorer la fonction cognitive ; ou
(f) comprenant également un ou plusieurs prébiotiques ou probiotiques.

6. Composition pour utilisation selon la revendication 5 ou utilisation selon la revendication 5, dans laquelle le CLON est l'arginine ou un dérivé libérant de l'oxyde nitrique de celle-ci.

7. Composition pour une utilisation selon la revendication 5 ou utilisation selon la revendication 5, dans laquelle la composition est une composition alimentaire comprenant en outre environ 15 % à environ 50 % de protéines, environ 5 % à environ 40 % de matière grasse, environ 5 % à environ 10 % de teneur en cendres, et ayant une teneur en humidité d'environ 5 % à environ 20 %.

8. Composition pour une utilisation selon l'une quelconque des revendications 1, 2, 5 à 7 ou utilisation selon l'une quelconque des revendications 3 à 7, dans laquelle la composition est une composition pharmaceutique ou nutraceutique comprenant un ou plusieurs supports, diluants, ou excipients acceptables sur le plan pharmaceutique ou nutraceutique.

9. Composition pour une utilisation selon l'une quelconque des revendications 1, 2, 5 à 8 ou utilisation selon l'une quelconque des revendications 3 à 8,
(a) dans laquelle la composition est une composition pharmaceutique ou nutraceutique qui comprend éventuellement un ou plusieurs médicaments cognitifs ; ou
(b) dans laquelle l'animal présente un phénotype associé à une déficience cognitive liée à l'âge ; éventuellement dans laquelle le phénotype comprend une ou plusieurs parmi une aptitude diminuée à se souvenir, une perte de mémoire à court terme, une vitesse d'apprentissage réduite, une aptitude affaiblie d'apprentissage, une diminution des aptitudes à résoudre les problèmes, une aptitude de concentration diminuée, une diminution de la performance motrice, une confusion accrue, ou la démence, en comparaison à un animal témoin ne présentant pas le phénotype.

10. Composition pour une utilisation selon l'une quelconque des revendications 1, 2, 5 à 9 ou utilisation selon l'une quelconque des revendications 3 à 9, dans laquelle l'animal est
(a) un humain ou un animal de compagnie ; ou
(b) un animal jeune ou adulte ; ou
(c) un animal vieillissant.

11. Composition pour utilisation selon l'une quelconque des revendications 1, 2, 5 à 9 ou utilisation selon l'une quelconque des revendications 3 à 9, dans laquelle l'animal est un animal vieillissant.

12. Lot appropriée pour administrer une composition comprenant d'environ 0,1 % à 30 % d'un ou plusieurs AGPCL, d'environ 1 % à 10 % de CLON et d'environ 1 % à 20 % de TCM à un animal comprenant dans récipients indépendants dans un conditionnement unique ou dans des récipients indépendants dans un conditionnement virtuel, comme il convient pour le composant de lot, (a) un ou plusieurs AGPCL, (b) un ou plusieurs CLON, (c) un ou plusieurs TCM (d) une ou plusieurs vitamines B et un ou plusieurs antioxydants et un ou plusieurs parmi (1) un ou plusieurs autres ingrédients propres à la consommation par un animal ; (2) un ou plusieurs médicaments cognitifs ; (3) un ou plusieurs prébiotiques ; (4) un ou plusieurs probiotiques ; (5) un ou plusieurs dispositifs de diagnostic appropriés pour déterminer si un animal pourrait bénéficier des compositions et procédés pour améliorer la fonction cognitive et des fonctions apparentées ; (6) des instructions sur la façon de combiner ou de préparer l'AGPCL, le CLON, et les TCM et n'importe quels autres ingrédients fournis dans le lot pour administration à un animal ; (7) des instructions sur la façon d'utiliser les composants combinés du lot, des composants préparés du lot, ou d'autres composants du lot pour le bienfait de l'animal ; et (8) un dispositif pour administrer les composants combinés ou préparés du lot à un animal.

13. Procédé de fabrication d'une composition alimentaire comprenant d'environ 0,1 % à 30 % d'AGPCL, d'environ 1 % à 10 % de CLON, d'environ 1 % à 20 % de TCM et un ou plusieurs ingrédients propres à la consommation par un animal comprenant le mélange d'un ou plusieurs ingrédients propres à la consommation par un animal avec de l'AGPCL, du CLON, et des TCM ou l'application d'AGPCL, de CLON, et des TCM séparément ou dans n'importe quelle combinaison sur la composition alimentaire ; dans lequel les ingrédients appropriés pour la consommation par un animal sont une ou plusieurs vitamines B et un ou plusieurs antioxydants.

14. Emballage contenant une composition selon la revendication 1, et une étiquette fixée à l'emballage contenant un mot ou des mots, une photo, un dessin, un acronyme, un slogan, une phrase, ou un autre dispositif, ou une combinaison de ceux-ci, qui indique que le contenu de l'emballage contient une composition appropriée pour améliorer la fonction cognitive ; altérer la fonction cognitive, motrice, ou comportementale ; réduire ou empêcher le déclin de l'interaction sociale ; réduire ou empêcher des changements comportementaux liés à l'âge ; augmenter la facilité d'apprentissage ; maintenir une fonction cérébrale optimale ; faciliter l'apprentissage et la mémoire ; et/ou réduire la perte de mémoire chez un animal.
